# EUROPEAN PATENT SPECIFICATION

(11) **EP 2 227 541 B1**
(45) Date of publication and mention of the grant of the patent: **19.06.2013**
(21) Application number: 08862527.2
(22) Date of filing: 18.12.2008
(51) Int. Cl.: C12N 15/09, C12N 9/26

(54) **RECOMBINANT MICROORGANISM HAVING AN ABILITY OF USING SUCROSE AS A CARBON SOURCE**
REKOMBINANTER MIKROORGANISMUS MIT FÄHIGKEIT ZUR VERWENDUNG VON SACCHAROSE ALS KOHLENSTOFFQUELLE
MICRO-ORGANISME RECOMBINANT POSSÉDANT LA CAPACITÉ D'UTILISER LE SACCHAROSE EN TANT QUE SOURCE DE CARBONE

(30) Priority: 18.12.2007 KR 20070133239; 28.10.2008 KR 20080106113
(43) Date of publication of application: 15.09.2010
(73) Proprietor: Korea Advanced Institute of Science and Technology, Daejeon 305-701 (KR)
(72) Inventor: LEE, Sang Yup, Daejeon 305-761 (KR); LEE, Jeong Wook, Daejeon 305-701 (KR); SONG, Hyohak, Daejeon 302-283 (KR); KIM, Ji Mahn, Seoul 135-110 (KR); CHOI, Sol, Jeju-si Jeju-do 690-012 (KR); PARK, Jin Hwan, Suwon-si Gyeonggi-do 443-773 (KR)
(74) Representative: Hiebl, Inge Elisabeth
(86) International application number: PCT/KR2008/007533
(87) International publication number: WO 2009/078687

(56) References cited:
- WO-A2-2007/041269
- DATABASE GENBANK "PtsG protein [Mannheimia succiniciproducens MBEL55E]", XP002672507, retrieved from NCBI Database accession no. AAU37391
- DATABASE EMBL [Online] 29 November 2007 (2007-11-29), "Mannheimia succiniciproducens MBEL55E, complete genome.", XP002672508, retrieved from EBI Database accession no. AE016827
- HONG SOON HO ET AL: "The genome sequence of the capnophilic rumen bacterium Mannheimia succiniciproducens", NATURE BIOTECHNOLOGY, vol. 22, no. 10, 1 October 2004 (2004-10-01), pages 1275-1281, XP002498825, NATURE PUBLISHING GROUP, NEW YORK, NY, US ISSN: 1087-0156, DOI: 10.1038/NBT1010 [retrieved on 2004-09-19]
- JAHREIS K ET AL: "Adaptation of sucrose metabolism in the Escherichia coli wild-type strain EC3132", JOURNAL OF BACTERIOLOGY, vol. 184, no. 19, 1 October 2002 (2002-10-01), pages 5307-5316, XP002984526, AMERICAN SOCIETY FOR MICROBIOLOGY, WASHINGTON, DC; US ISSN: 0021-9193, DOI: 10.1128/JB.184.19.5307-5316.2002
- JANG Y S ET AL: "Identification of sucrose uptake system in Mannheimia succiniciproducens", NEW BIOTECHNOLOGY, vol. 25, no. Supp., 1 September 2009 (2009-09-01), page S274, XP026461629, ELSEVIER BV, NL ISSN: 1871-6784, DOI: 10.1016/J.NBT.2009.06.617 [retrieved on 2009-08-12]
- LEE J W ET AL: "A serial phosphotransferase system for sucrose utilization in Mannheimia succiniciproducens and its use in the enhanced succinate production", JOURNAL OF BIOTECHNOLOGY, vol. 150, no. Supp., 1 November 2010 (2010-11-01), page 350, XP027489873, ELSEVIER SCIENCE PUBLISHERS, AMSTERDAM, NL ISSN: 0168-1656, DOI: 10.1016/J.JBIOTEC.2010.09.390 [retrieved on 2010-11-01]
- LEE J W ET AL: "Metabolic and evolutionary engineering of Mannheimia succiniciproducens for the enhanced succinate productivity", JOURNAL OF BIOTECHNOLOGY, vol. 150, no. Supp., 1 November 2010 (2010-11-01), page 516, XP027490304, ELSEVIER SCIENCE PUBLISHERS, AMSTERDAM, NL ISSN: 0168-1656, DOI: 10.1016/J.JBIOTEC.2010.09.821 [retrieved on 2010-11-01]
- DATABASE GENBANK 'PtsG protein [Mannheimia succiniciproducens MBEL55E]' Database accession no. AAU37391.1
- DATABASE GENBANK 'SacC protein [Mannheimia succiniciproducens MBEL55E]', XP008136746 Database accession no. AAU37516.1
- DATABASE GENBANK 'sucrose hydrolase [Escherichia coli]' Database accession no. AAP795D3.1
- DATABASE GENBANK 'sucrase-6-phosphate hydrolase [Bacillus subtilis]', XP008136747 Database accession no. CAB15830.1
- DATABASE GENBANK 'Mannheimia succiniciproducens MBEL55E' Database accession no. AE016827.1
- DATABASE GENBANK 'E. coli anion symport for sucrose (cscB), fructokinase (cscK), and sucrose hydrolase (cscA) genes', XP008136751 Database accession no. AY314757.1
- DATABASE GENBANK 'subtilis genomic region', XP008136745 Database accession no. X73124.1
- SOON HO HONG ET AL.: 'The genome sequence of the capnophilic rumen bacterium Mannheimia succiniciproducens' NATURE BIOTECHNOLOGY vol. 22, no. 10, October 2004, pages 1275 - 1281, XP002498825
- MIN-WOO MOON ET AL.: 'Analysis of enzyme II gene mutants for sugar transport and heterologous expression of fructokinase gene in Corynebaacterium glutamicum ATCC 13032' FEMS MICROBIOLOGY LETTERS vol. 244, 2005, pages 259 - 266, XP025394333
- KNUT JAHREIS ET AL.: 'Adaptation of sucrose metabolism in the Escherichia coli wild-type strain EC3132' JOURNAL OF BACTERIOLOGY vol. 184, no. 19, October 2002, pages 5307 - 5316, XP002984526

### SEQUENCE LISTING

<110> KOREA ADVANCED INSTITUTE OF SCIENCE AND TECHNOLOGY
<120> Recombinant Microorganism Having an Ability of Using Sucrose as a Carbon Source
<130> 21522EP
<140> EP 08 862 527.2
   <141> 2008-12-18
<150> KR 10-2007-0133239
   <151> 2007-12-18
<150> KR.10-2008-0106113
   <151> 2008-10-28
<160> 52
<170> Patent In version 3.2
<210> 1
   <211> 492
   <212> PRT
   <213> Mannheimia succiniciproducens MBEL55E
<200>
   <223> Sucrose phosphotransferase (PtsG)
<400> 1
   Met Leu Val Leu Ala Arg Ile Gly Glu Asn Phe Cys Leu Ile Tyr Lys
   1               5                   10                  15
   Arg Gly Val Ala Met Asn Tyr Pro Lys Ile Ala Gln Gln Val Ile Glu
               20                  25                  30
   Lys Leu Gly Gly Lys Glu Asn Ile Ala Asn Leu Ala His Cys Ala Thr
           35                  40                  45
   Arg Leu Arg Leu Thr Met Asn Asp Glu Ser Lys Ile Asp Lys Gln Ala
       50                  55                  60
   Ile Glu Asp Ile Glu Gly Val Lys Gly Gln Phe Ser Thr Ser Gly Gln
   65                  70                  75                  80
   Tyr Gln Ile Ile Phe Gly Ser Gly Thr Val Asn Lys Val Tyr Ala Glu
                   85                  90                  95
   Met Asn Thr Ile Met Asn Gly Ser Pro Ser Ala Asp Ser Thr Gly Glu
               100                 105                 110
   Ser Gln Gln Ala Lys Gly Pro Gln Gln Gly Leu Ile Gln Arg Leu Ile
           115                 120                 125
   Lys Gly Leu Ala Asp Ile Phe Val Pro Ile Ile Pro Ala Ile Val Ala
       130                 135                 140
   Gly Gly Leu Leu Met Gly Ile Asn Asn Val Phe Thr Ala Lys Asp Leu
   145                 150                 155                 160
   Phe Glu Glu Gly Arg Thr Leu Leu Asp Leu Tyr Pro Gln Tyr Lys Asp
                   165                 170                 175
   Leu Ala Asp Leu Ile Asn Thr Phe Ala Asn Ala Pro Phe Val Phe Leu
               180                 185                 190
   Pro Val Leu Leu Gly Phe Ser Ala Thr Arg Lys Phe Gly Gly Asn Pro
           195                 200                 205
   Phe Leu Gly Ala Thr Leu Gly Met Leu Leu Val His Pro Ala Leu Thr
       210                 215                 220
   Asn Ala Tyr Gly Tyr Ala Glu Ala Leu Ala Gly Gly Asn Leu Gln Leu
   225                 230                 235                 240
   Trp Asn Ile Phe Gly Leu Glu Ile Glu Lys Val Gly Tyr Gln Gly Thr
                   245                 250                 255
   Val Ile Pro Val Leu Ile Ala Ala Trp Val Leu Ala Thr Leu Glu Lys
               260                 265                 270
   Phe Leu Val Lys Val Val Pro Ser Val Leu Asn Asn Leu Val Thr Pro
           275                 280                 285
   Leu Phe Ser Leu Phe Ile Thr Gly Phe Leu Ala Phe Thr Val Ile Gly
       290                 295                 300
   Pro Phe Gly Arg Glu Ala Gly Glu Phe Leu Ser Gln Gly Leu Thr Trp
   305                 310                 315                 320
   Leu Tyr Asp Thr Leu Gly Phe Ile Gly Gly Gly Val Phe Gly Ala Leu
                   325                 330                 335
   Tyr Ala Pro Ile Val Ile Thr Gly Met His Gln Thr Phe Ile Ala Ile
               340                 345                 350
   Glu Thr Gln Leu Leu Ala Ser Thr Ala Ala Thr Phe Ile Phe Pro Ile
           355                 360                 365
   Ala Ala Met Ser Asn Ile Ala Gln Gly Ala Ala Cys Leu Ala Val Ala
       370                 375                 380
   Val Leu Asn Lys Asp Ala Lys Thr Arg Gly Leu Ala Leu Pro Ser Gly
   385                 390                 395                 400
   Ile Ser Ala Leu Leu Gly Ile Thr Glu Pro Ala Met Phe Gly Val Asn
                   405                 410                 415
   Leu Arg Phe Arg Tyr Pro Phe Tyr Ala Ala Met Leu Gly Ala Gly Ser
               420                 425                 430
   Ala Ala Ala Phe Ile Ala Phe Phe Asn Val Lys Ala Thr Ala Leu Gly
           435                 440                 445
   Ala Ala Gly Leu Ile Gly Ile Ala Ser Ile Arg Ala Gly Asp Trp Gly
       450                 455                 460
   Met Tyr Ser Val Gly Met Val Ile Ser Phe Cys Val Ala Phe Ala Ala
   465                 470                 475                 480
   Ala Leu Val Leu Gly Ala Arg Ala Asn Ala Lys Glu
                   485                 490
<210> 2
   <211> 1479
   <212> DNA
   <213> Mannheimia succiniciproducens MBEL55E
<200>
   <223> Gene encoding sucrose phosphotranferase (ptsG)
<400> 2
   ttgctcgttt tagctagaat tggcgaaaat ttttgcttaa tttataaacg aggagtcgct      60
   atgaactacc ctaaaattgc ccaacaggtt atcgaaaaac ttggcggaaa agaaaatatc     120
   gctaatcttg cgcattgtgc aacgcgtttg cgcttgacaa tgaatgacga aagtaaaatc     180
   gacaaacagg ccattgaaga tatcgagggc gtaaaagggc agttttcaac ctccggtcaa     240
   taccaaatta ttttcggttc aggtacggtg aataaagttt acgccgaaat gaataccatt     300
   atgaacggtt cgccgtcggc ggattccacc ggggaaagtc aacaggcgaa agggccgcag     360
   caaggtttga ttcaacgatt aattaaaggt ctggctgata ttttcgttcc cattattccg     420
   gctattgtcg ccggcggttt gttaatgggg attaataatg tctttaccgc aaaagattta     480
   ttcgaagaag ggaggacatt actcgacctt tatccgcaat acaaagattt agcggattta     540
   attaatacct ttgctaacgc gccttttgtg tttctgcccg tattgttagg tttctcggca     600
   accagaaaat tcggtggcaa tccgttctta ggagcgacat taggtatgtt gctcgttcac     660
   cccgctttaa ccaatgctta cggttatgcg gaagcgttag ccggcggcaa tcttcaatta     720
   tggaatattt tcgggctaga gattgaaaaa gtcggttatc aaggtacggt tattcccgtt     780
   ttaattgccg cctgggtatt ggcgacttta gaaaaattct tagtgaaagt agtgccttcc     840
   gtattaaata atttagtcac gccgttattt tcattattta tcaccggttt tttggctttc     900
   accgtaatcg gacctttcgg tcgtgaagcg ggggaatttt taagtcaggg tttaacctgg     960
   ttatatgata ctttaggttt tatcggcggc ggcgtgttcg gcgcattata cgcacctatc    1020
   gtgattaccg gtatgcacca aacctttatc gccattgaaa cgcaattgct ggcaagcact    1080
   gcggcaactt ttatcttccc gattgccgcc atgtcgaata ttgcgcaggg tgccgcttgt    1140
   ttagccgttg ccgtgttaaa taaagatgcc aaaacccgag gtctggcgtt gccttccggt    1200
   atttccgcat tattagggat taccgaacct gccatgttcg ggtgaatttt gcgcttccgt     1260
   tatccgttct atgcggctat gttaggtgcc ggttccgccg cggcgtttat cgcgttcttc    1320
   aatgttaaag ccactgcgct tggcgcggcg ggcttaatcg gtatcgcatc aattcgtgcc    1380
   ggcgactggg gaatgtattc cgtggggatg gtaatttcgt tttgtgtggc tttcgctgcg    1440
   gcattagtgc tgggcgcaag agctaacgca aaagaatag                           1479
<210> 3
   <211> 502
   <212> PRT
   <213> Mannheimia succiniciproducens MBEL55E
<200>
   <223> Sucrose-6-phosphate hydrolase (SacC)
<400> 3
   Met Arg Ser Phe Leu Pro His Phe Ser Leu Phe Tyr Phe His Gln Gly
   1               5                   10                  15
   Ile Met Met Ile Ile Phe Asn Asn Gly Lys Tyr Lys Ser Ile Leu Ala
               20                  25                  30
   Ala Glu Gln Gly Glu Leu Glu Arg Ile Lys Ser Glu Val Glu Lys Asp
           35                  40                  45
   Arg Asp Phe Arg Pro Tyr Tyr His Leu Ala Pro Ser Thr Gly Leu Leu
       50                  55                  60
   Asn Asp Pro Asn Gly Leu Val Phe Asp Gly Glu Lys Phe His Leu Phe
   65                  70                  75                  80
   Tyr Gln Trp Phe Pro Phe Asp Ala Ile His Gly Met Lys His Trp Lys
                   85                  90                  95
   His Phe Thr Thr Glu Asp Phe His Ile Tyr Thr Glu Ala Asp Pro Leu
               100                 105                 110
   Ile Pro Cys Glu Leu Phe Glu Ser His Gly Cys Tyr Ser Gly Gly Ala
           115                 120                 125
   Leu Pro Val Gly Asp Lys Ile Ala Ala Phe Tyr Thr Gly Asn Thr Arg
       130                 135                 140
   Arg Ala Ala Asp Asn Gln Arg Val Pro Phe Gln Asn Leu Ala Ile Phe
   145                 150                 155                 160
   Asp Arg Thr Gly Lys Leu Leu Ser Lys Arg Pro Leu Ile Glu Asn Ala
                   165                 170                 175
   Pro Lys Gly Tyr Thr Glu His Val Arg Asp Pro Lys Pro Tyr Phe Thr
               180                 185                 190
   Lys Glu Gly Lys Ile Arg Phe Ile Cys Gly Ala Gln Arg Glu Asp Leu
           195                 200                 205
   Thr Gly Thr Ala Ile Ile Phe Glu Met Asp Asn Leu Asp Asp Glu Pro
       210                 215                 220
   Arg Leu Leu Gly Glu Leu Ser Leu Pro Ala Phe Asp Asn Gln Lys Val
   225                 230                 235                 240
   Phe Met Trp Glu Cys Pro Asp Leu Leu Lys Val Gly Asp Asn Asp Ile
                   245                 250                 255
   Phe Ile Trp Ser Pro Gln Gly Lys Arg Arg Glu Ala Arg Arg Phe Gln
               260                 265                 270
   Asn Asn Phe His Ala Val Tyr Ala Val Gly Lys Leu Asp Asp Arg Thr
           275                 280                 285
   Phe Asn Ala Ala His Ile Ala Glu Leu Asp Gln Gly Phe Asp Phe Tyr
       290                 295                 300
   Ala Pro Gln Thr Phe Ala Gly Leu Glu Asn Gln Lys His Ala Val Met
   305                 310                 315                 320
   Phe Gly Trp Cys Gly Met Pro Asp Leu Thr Tyr Pro Thr Asp Lys Tyr
                   325                 330                 335
   Lys Trp His Ser Met Leu Thr Leu Pro Arg Glu Ile Thr Leu Gln Gly
               340                 345                 350
   Asn Arg Leu Val Gln Arg Pro Ile Lys Glu Ile Tyr Gln Asn Leu Thr
           355                 360                 365
   Ala Leu Ser Gln Ile Ser Leu Gln Gln Gln Ala Glu Ile Gln Asp Leu
       370                 375                 380
   Asp Arg Ala Tyr Ile Lys Phe Asp Ala Glu Asn Thr Ala Phe Asn Ile
   385                 390                 395                 400
   Arg Phe Phe Ala Asn Glu Gln Gly Gln Thr Leu Ser Leu Ser Tyr Asp
                   405                 410                 415
   Gly Glu Leu Val Cys Leu Asp Arg Ser Gln Thr Glu Glu Thr Glu Trp
               420                 425                 430
   Met Lys Lys Phe Ala Ser Gln Arg Tyr Cys Glu Ile Lys Asn Leu Arg
           435                 440                 445
   Gln Val Glu Ile Phe Phe Asp Arg Ser Ile Ile Glu Ile Phe Leu Asn
       450                 455                 460
   Asp Gly Glu Lys Ala Leu Thr Ser Arg Phe Phe Ile Ala Asn Arg Gln
   465                 470                 475                 480
   Asn Ser Val Lys Thr Asp Arg Thr Leu Arg Leu Asn Val Gly Tyr Pro
                   485                 490                 495
   Lys Glu Ile Glu Tyr Lys
               500
<210> 4
   <211> 1509
   <212> DNA
   <213> Mannheimia succiniciproducens MBEL55E
<200>
   <223> Gene encoding sucrose-6-phosphate hydrolase (SacC)
<400> 4
   gtgcggtcgt ttttaccgca cttttcttta ttttattttc accaaggtat aatgatgatt      60
   atatttaata acggtaaata taaaagcatt ttggcggccg aacagggcga gcttgaacga     120
   attaaaagcg aggtagaaaa agatcgggat tttcgcccct actaccatct cgcgccatct     180
   acaggcttac taaacgatcc caacggtttg gtttttgacg gcgaaaaatt tcatctgttc     240
   tatcaatggt tcccgtttga tgccattcac ggcatgaaac actggaagca tttcacgacc     300
   gaggattttc atatctatac cgaagccgat ccgcttatcc cttgcgaact ttttgaaagt     360
   cacggttgtt attccggcgg cgccttacca gtgggcgata aaatcgccgc attttatacc     420
   ggtaacacaa gacgcgctgc ggataaccaa cgggttccct ttcaaaattt agcgattttt     480
   gaccgcaccg gtaaacttct cagtaaacgc ccattaattg aaaatgcacc gaaaggctac     540
   accgaacacg ttcgtgatcc gaaaccttac ttcacaaaag aaggaaaaat ccgttttatt     600
   tgcggcgcac aacgtgaaga tttaaccggc accgccatta tttttgaaat ggataatctt     660
   gatgatgagc cgcgcttatt aggcgaattg tctctccccg cttttgataa tcaaaaggtg     720
   tttatgtggg aatgcccgga tttattgaaa gtcggcgata acgatatttt catctggtct     780
   ccgcaaggca aacggcgcga agcccgccgg ttccaaaata attttcatgc ggtctatgcc     840
   gtaggaaaat tggatgatcg gacatttaat gccgctcata ttgccgaact tgatcaaggt     900
   ttcgatttct atgcgccgca aacttttgcc ggcctggaaa atcaaaagca tgccgttatg     960
   ttcggttggt gcggtatgcc ggatttgacc tacccgacgg ataaatacaa atggcattca    1020
   atgctgactc tcccgcggga aattacattg caaggcaata ggcttgttca gcgcccgata    1080
   aaagaaattt atcaaaattt gaccgcactt tcgcaaattt ccctgcaaca gcaagcggaa    1140
   attcaggatt tagatcgagc ctatattaaa tttgacgcgg aaaacacagc gtttaatatc    1200
   cgcttttttg ccaacgaaca aggacaaacg ctctcgcttt cttatgacgg agaacttgtt    1260
   tgtctggatc gttcgcaaac ggaagaaacg gaatggatga aaaaatttgc tagtcagcgt    1320
   tattgtgaaa taaagaatct gcgacaagtg gaaattttct ttgaccgctc aattattgag    1380
   attttcctga atgacggcga aaaagccctg acttcgagat tctttattgc gaaccgccaa    1440
   aattccgtca aaaccgaccg cactttgcgg ttaaacgtcg gttatccgaa agaaattgaa    1500
   tataaatag                                                            1509
<210> 5
   <211> 310
   <212> PRT
   <213> Mannheimia succiniciproducens MBEL55E
<200>
   <223> Fructokinase (RbsK)
<400> 5
   Met His Met Thr Asn Lys Ile Trp Val Leu Gly Asp Ala Val Val Asp
   1               5                   10                  15
   Leu Ile Pro Asp Gly Asp Asn His Tyr Leu Arg Cys Ala Gly Gly Ala
               20                  25                  30
   Pro Ala Asn Val Ala Val Gly Val Ala Arg Leu Gly Val Pro Ser Ala
           35                  40                  45
   Phe Ile Gly Arg Val Gly Lys Asp Pro Leu Gly Glu Phe Met Arg Asp
       50                  55                  60
   Thr Leu Asn Gln Glu Asn Val Asn Thr Asp Tyr Met Leu Leu Asp Pro
   65                  70                  75                  80
   Lys Gln Arg Thr Ser Thr Val Val Val Gly Leu Thr Asp Gly Glu Arg
                   85                  90                  95
   Ser Phe Thr Phe Met Val Asn Pro Ser Ala Asp Gln Phe Leu Gln Ile
               100                 105                 110
   Ser Asp Leu Pro Gln Phe Gln Ala Gly Asp Trp Leu His Cys Cys Ser
           115                 120                 125
   Ile Ala Leu Ile Asn Glu Pro Thr Arg Ser Ala Thr Phe Thr Ala Met
       130                 135                 140
   Lys Asn Ile Arg Ala Ala Gly Gly Lys Val Ser Phe Asp Pro Asn Leu
   145                 150                 155                 160
   Arg Glu Ser Leu Trp Lys Ser Gln Asp Glu Met Ile Asp Val Val Met
                   165                 170                 175
   Glu Ala Val Ser Leu Ala Asp Val Leu Lys Phe Ser Glu Glu Glu Leu
               180                 185                 190
   Thr Leu Leu Thr His Thr Asp Ser Leu Glu Lys Ser Phe Glu Lys Ile
           195                 200                 205
   Thr Ala Leu Tyr Pro Asp Lys Leu Ile Ile Val Thr Leu Gly Lys Glu
       210                 215                 220
   Gly Ala Leu Tyr His Leu His Gly Lys Lys Glu Val Val Ala Gly Lys
   225                 230                 235                 240
   Ala Leu Lys Pro Val Asp Thr Thr Gly Ala Gly Asp Ala Phe Val Ser
                   245                 250                 255
   Gly Leu Leu Ala Gly Leu Ser Gln Thr Glu Asn Trp Gln Gln Pro Glu
               260                 265                 270
   Gln Leu Val Thr Ile Ile Arg Gln Ala Asn Ala Ser Gly Ala Leu Ala
           275                 280                 285
   Thr Thr Ala Lys Gly Ala Met Ser Ala Leu Pro Asn Gln Gln Gln Leu
       290                 295                 300
   Ala Glu Phe Leu Ala Asn
   305                 310
<210> 6
   <211> 933
   <212> DNA
   <213> Mannheimia succiniciproducens MBEL55E
<200>
   <223> Gene encoding fructokinase (rbsK)
<400> 6
   atgcatatga caaacaaaat ttgggtatta ggcgatgccg tggtggattt aattcctgac     60
   ggagacaacc attatttgcg ttgcgcaggc ggcgcaccgg ctaatgtggc ggtcggcgtt    120
   gcccgtttag gtgtgcctag cgcatttatc ggccgtgtag gtaaagatcc gttaggggaa    180
   tttatgcgcg atacgctgaa tcaggaaaat gtaaacaccg attatatgtt gttagatcct    240
   aaacaacgta cttcgacggt ggtggttgga ttaactgacg gcgaacgtag ttttaccttt    300
   atggtgaatc caagtgcgga tcaattttta caaatttccg atctgccgca atttcaagcc    360
   ggagactggt tgcactgctg ctctatcgcc ttaatcaatg aaccgacccg cagcgctact    420
   ttcacggcaa tgaaaaatat ccgtgcggcc ggcggtaaag tatctttcga tccgaattta    480
   cgcgaaagct tatggaaatc ccaggatgaa atgatcgatg tggtgatgga agcggtaagc    540
   cttgccgacg tattgaaatt ttcagaagaa gaattaacgc tgttaaccca taccgacagc    600
   ctggaaaaat cttttgaaaa aatcaccgca ctttatcccg ataaattgat tattgtcact    660
   ttagggaaag aaggtgcgct ctatcatctg cacggtaaaa aagaggtggt tgcagggaaa    720
   gcgctgaaac cggtagatac caccggggcc ggcgacgctt ttgtcagcgg gttattagcc    780
   ggattatcac aaacggaaaa ctggcagcaa cctgaacaac tcgttactat tattcgccag    840
   gccaacgcca gcggcgcgct tgccacaacg gcaaaaggcg ctatgtcggc attaccgaat    900
   cagcaacaat tagcggaatt tttagcaaac taa                                 933
<210> 7
   <211> 477
   <212> PRT
   <213> E. coli W
<200>
   <223> Sucrose hydrolase (CscA)
<400> 7
   Met Thr Gln Ser Arg Leu His Ala Ala Gln Asn Ala Leu Ala Lys Leu
   1               5                   10                  15
   His Glu His Arg Gly Asn Thr Phe Tyr Pro His Phe His Leu Ala Pro
               20                  25                  30
   Pro Ala Gly Trp Met Asn Asp Pro Asn Gly Leu Ile Trp Phe Asn Asp
           35                  40                  45
   Arg Tyr His Ala Phe Tyr Gln His His Pro Met Ser Glu His Trp Gly
       50                  55                  60
   Pro Met His Trp Gly His Ala Thr Ser Asp Asp Met Ile His Trp Gln
   65                  70                  75                  80
   His Glu Pro Ile Ala Leu Ala Pro Gly Asp Asp Asn Asp Lys Asp Gly
                   85                  90                  95
   Cys Phe Ser Gly Ser Ala Val Asp Asp Asn Gly Val Leu Ser Leu Ile
               100                 105                 110
   Tyr Thr Gly His Val Trp Leu Asp Gly Ala Gly Asn Asp Asp Ala Ile
           115                 120                 125
   Arg Glu Val Gln Cys Leu Ala Thr Ser Arg Asp Gly Ile His Phe Glu
       130                 135                 140
   Lys Gln Gly Val Ile Leu Thr Pro Pro Glu Gly Ile Met His Phe Arg
   145                 150                 155                 160
   Asp Pro Lys Val Trp Arg Glu Ala Asp Thr Trp Trp Met Val Val Gly
                   165                 170                 175
   Ala Lys Asp Pro Gly Asn Thr Gly Gln Ile Leu Leu Tyr Arg Gly Ser
               180                 185                 190
   Ser Leu Arg Glu Trp Thr Phe Asp Arg Val Leu Ala His Ala Asp Ala
           195                 200                 205
   Gly Glu Ser Tyr Met Trp Glu Cys Pro Asp Phe Phe Ser Leu Gly Asp
       210                 215                 220
   Gln His Tyr Leu Met Phe Ser Pro Gln Gly Met Asn Ala Glu Gly Tyr
   225                 230                 235                 240
   Ser Tyr Arg Asn Arg Phe Gln Ser Gly Val Ile Pro Gly Met Trp Ser
                   245                 250                 255
   Pro Gly Arg Leu Phe Ala Gln Ser Gly His Phe Thr Glu Leu Asp Asn
               260                 265                 270
   Gly His Asp Phe Tyr Ala Pro Gln Ser Phe Leu Ala Lys Asp Gly Arg
           275                 280                 285
   Arg Ile Val Ile Gly Trp Met Asp Met Trp Glu Ser Pro Met Pro Ser
       290                 295                 300
   Lys Arg Glu Gly Trp Ala Gly Cys Met Thr Leu Ala Arg Glu Leu Ser
   305                 310                 315                 320
   Glu Ser Asn Gly Lys Leu Leu Gln Arg Pro Val His Glu Ala Glu Ser
                   325                 330                 335
   Leu Arg Gln Gln His Gln Ser Val Ser Pro Arg Thr Ile Ser Asn Lys
               340                 345                 350
   Tyr Val Leu Gln Glu Asn Ala Gln Ala Val Glu Ile Gln Leu Gln Trp
           355                 360                 365
   Ala Leu Lys Asn Ser Asp Ala Glu His Tyr Gly Leu Gln Leu Gly Thr
       370                 375                 380
   Gly Met Arg Leu Tyr Ile Asp Asn Gln Ser Glu Arg Leu Val Leu Trp
   385                 390                 395                 400
   Arg Tyr Tyr Pro His Glu Asn Leu Asp Gly Tyr Arg Ser Ile Pro Leu
                   405                 410                 415
   Pro Gln Arg Asp Thr Leu Ala Leu Arg Ile Phe Ile Asp Thr Ser Ser
               420                 425                 430
   Val Glu Val Phe Ile Asn Asp Gly Glu Ala Val Met Ser Ser Arg Ile
           435                 440                 445
   Tyr Pro Gln Pro Glu Glu Arg Glu Leu Ser Leu Tyr Ala Ser His Gly
       450                 455                 460
   Val Ala Val Leu Gln His Gly Ala Leu Trp Leu Leu Gly
   465                 470                 475
<210> 8
   <211> 1434
   <212> DNA
   <213> E. coli W
<200>
   <223> Gene encoding succrose hydrolase (CscA)
<400> 8
   atgacgcaat ctcgattgca tgcggcgcaa aacgccctag caaaacttca tgagcaccgg     60
   ggtaacactt tctatcccca ttttcacctc gcgcctcctg ccgggtggat gaacgatcca    120
   aacggcctga tctggtttaa cgatcgttat cacgcgtttt atcaacatca tccgatgagc    180
   gaacactggg ggccaatgca ctggggacat gccaccagcg acgatatgat ccactggcag    240
   catgagccta ttgcgctagc gccaggagac gataatgaca aagacgggtg tttttcaggt    300
   agtgctgtcg atgacaatgg tgtcctctca cttatctaca ccggacacgt ctggctcgat    360
   ggtgcaggta atgacgatgc aattcgcgaa gtacaatgtc tggctaccag tcgggatggt    420
   attcatttcg agaaacaggg tgtgatcctc actccaccag aaggaatcat gcacttccgc    480
   gatcctaaag tgtggcgtga agccgacaca tggtggatgg tagtcggggc gaaagatcca    540
   ggcaacacgg ggcagatcct gctttatcgc ggcagttcgt tgcgtgaatg gaccttcgat    600
   cgcgtactgg cccacgctga tgcgggtgaa agctatatgt gggaatgtcc ggactttttc    660
   agccttggcg atcagcatta tctgatgttt tccccgcagg gaatgaatgc cgagggatac    720
   agttaccgaa atcgctttca aagtggcgta atacccggaa tgtggtcgcc aggacgactt    780
   tttgcacaat ccgggcattt tactgaactt gataacgggc atgactttta tgcaccacaa    840
   agctttttag cgaaggatgg tcggcgtatt gttatcggct ggatggatat gtgggaatcg    900
   ccaatgccct caaaacgtga aggatgggca ggctgcatga cgctggcgcg cgagctatca    960
   gagagcaatg gcaaacttct acaacgcccg gtacacgaag ctgagtcgtt acgccagcag   1020
   catcaatctg tctctccccg cacaatcagc aataaatatg ttttgcagga aaacgcgcaa   1080
   gcagttgaga ttcagttgca gtgggcgctg aagaacagtg atgccgaaca ttacggatta   1140
   cagctcggca ctggaatgcg gctgtatatt gataaccaat ctgagcgact tgttttgtgg   1200
   cggtattacc cacacgagaa tttagacggc taccgtagta ttcccctccc gcagcgtgac   1260
   acgctcgccc taaggatatt tatcgataca tcatccgtgg aagtatttat taacgacggg   1320
   gaagcggtga tgagtagtcg aatctatccg cagccagaag aacgggaact gtcgctttat   1380
   gcctcccacg gagtggctgt gctgcaacat ggagcactct ggctactggg ttaa         1434
<210> 9
   <211> 480
   <212> PRT
   <213> Bacillus subtilis
<200>
   <223> Sucrose-6-phosphate hydrolase (SacA)
<400> 9
   Met Thr Ala His Asp Gln Glu Leu Arg Arg Arg Ala Tyr Glu Glu Val
   1               5                   10                  15
   Glu Lys Lys Glu Pro Ile Ala Asn Ser Asp Pro His Arg Gln His Phe
               20                  25                  30
   His Ile Met Pro Pro Val Gly Leu Leu Asn Asp Pro Asn Gly Val Ile
           35                  40                  45
   Tyr Trp Lys Gly Ser Tyr His Val Phe Phe Gln Trp Gln Pro Phe Gln
       50                  55                  60
   Thr Gly His Gly Ala Lys Phe Trp Gly His Tyr Thr Thr Gln Asp Val
   65                  70                  75                  80
   Val Asn Trp Lys Arg Glu Glu Ile Ala Leu Ala Pro Ser Asp Trp Phe
                   85                  90                  95
   Asp Lys Asn Gly Cys Tyr Ser Gly Ser Ala Val Thr Lys Asp Asp Arg
               100                 105                 110
   Leu Tyr Leu Phe Tyr Thr Gly Asn Val Arg Asp Gln Asp Gly Asn Arg
           115                 120                 125
   Glu Thr Tyr Gln Cys Leu Ala Val Ser Asp Asp Gly Leu Ser Phe Glu
       130                 135                 140
   Lys Lys Gly Val Val Ala Arg Leu Pro Glu Ala Ile Leu Thr Ala His
   145                 150                 155                 160
   Phe Ser Arg Ser Glu Val Trp Glu His Glu Gly Thr Trp Tyr Met Val
                   165                 170                 175
   Ile Gly Ala Gln Thr Glu Asn Leu Lys Gly Gln Ala Val Leu Phe Ala
               180                 185                 190
   Ser Asp Asn Leu Thr Glu Trp Arg Phe Leu Gly Pro Ile Thr Gly Ala
           195                 200                 205
   Gly Phe Asn Gly Leu Asp Asp Phe Gly Tyr Met Trp Glu Cys Pro Asp
       210                 215                 220
   Leu Phe Ser Leu Gln Gly Ser Asp Val Leu Ile Val Ser Pro Gln Gly
   225                 230                 235                 240
   Leu Glu Ala Asp Gly Phe Arg Tyr Gln Asn Val Tyr Gln Ser Gly Tyr
                   245                 250                 255
   Phe Val Gly Arg Leu Asp Tyr Asn Lys Pro Glu Leu Lys His Gly Glu
               260                 265                 270
   Phe Thr Glu Leu Asp Gln Gly Phe Asp Phe Tyr Ala Pro Gln Thr Leu
           275                 280                 285
   Glu Asp Asp Gln Gly Arg Arg Ile Leu Phe Ala Trp Met Ala Val Pro
       290                 295                 300
   Asp Gln Asp Glu Gly Ser His Pro Thr Ile Asp Cys His Trp Ile His
   305                 310                 315                 320
   Cys Met Thr Leu Pro Arg Gln Leu Thr Leu Ser Gly Gln Lys Leu Ile
                   325                 330                 335
   Gln Gln Pro Leu Pro Glu Leu Lys Ala Met Arg Arg Asn Glu Lys Lys
               340                 345                 350
   Ile His Ile Asn Met His Gly Ser Ser Gly Ala Leu Pro Val Glu Lys
           355                 360                 365
   Pro Glu Arg Thr Glu Ile Leu Leu Glu Asp Ile His Thr Glu Ser Gly
       370                 375                 380
   Phe Ser Ile Ser Ile Arg Gly Thr Ala Thr Phe Ser Phe His Lys Asp
   385                 390                 395                 400
   Glu Gly Ile Val Thr Leu Glu Arg Lys Ser Phe Asp Gly Lys Arg Thr
                   405                 410                 415
   Glu Ala Arg His Cys Arg Ile Lys Asp Leu His Thr Val His Met Phe
               420                 425                 430
   Leu Asp Ala Ser Ser Val Glu Ile Phe Ile Asn Asn Gly Glu Glu Val
           435                 440                 445
   Phe Ser Ala Arg Tyr Phe Pro Phe Pro Gly Asn His Glu Val Thr Ala
       450                 455                 460
   Ser Ala Thr Gly Lys Ser Glu Met Asn Val Gly Ile Trp Thr Leu Met
   465                 470                 475                 480
<210> 10
   <211> 1443
   <212> DNA
   <213> Bacillus subtilis
<200>
   <223> Gene encoding sucrose-6-phosphate hydrolase (SacA)
<400> 10
   atgacagcac atgaccagga gcttcgtcgc cgggcttatg aagaagtgga gaaaaaagag     60
   cccatcgcta acagcgatcc gcaccgccag cattttcata tcatgccgcc ggttgggctg    120
   ctgaatgacc cgaatggcgt gatttattgg aagggcagct atcatgtatt ctttcagtgg    180
   cagccgtttc agacggggca cggcgcaaaa ttttgggggc attatacgac acaggatgtt    240
   gtgaattgga agcgggaaga gattgcgctg gctccgagtg attggtttga taaaaacggc    300
   tgctactcgg gcagcgctgt cacgaaagac gatcggctct atctttttta cacaggaaat    360
   gtcagggatc aggatggaaa tcgggaaacg tatcaatgcc ttgctgtttc tgacgacggg    420
   ctgtcctttg agaaaaaggg tgtcgtcgcc cgccttccgg aagcgatatt aacggcgcac    480
   ttttcgcgat ccgaagtatg ggagcatgaa ggcacatggt atatggtgat tggtgcgcaa    540
   acagagaatt tgaaagggca ggctgtgttg tttgcttctg ataacctgac agagtggaga    600
   tttcttggcc cgataaccgg cgcgggcttc aacgggctgg acgattttgg atacatgtgg    660
   gaatgccctg atttgttttc ccttcaagga tcggatgtgc tgattgtttc gcctcaaggg    720
   cttgaggctg acggtttccg ttatcagaac gtatatcaat caggttattt tgtcggccgc    780
   ctcgattata acaagcctga actgaagcat ggtgaattta cggagcttga tcaaggtttt    840
   gatttttacg cgccgcaaac acttgaagac gatcagggaa ggcggatttt atttgcatgg    900
   atggcggtgc ctgatcagga tgaagggtcc catccgacca ttgactgcca ctggattcac    960
   tgcatgacgc tgccgagaca gctgacgctt tcaggacaga agctgattca gcagccgctg   1020
   cctgagctaa aagccatgcg cagaaatgag aaaaaaatac acatcaacat gcatggatca   1080
   tctggtgcgc ttccagtgga aaaacctgaa agaactgaga ttctactgga agacattcat   1140
   acggagtctg gcttttcaat cagtatccgc ggaacggcta cgttttcctt ccataaagac   1200
   gaggggattg ttacgctgga acgaaagagc tttgacggaa aaagaacaga agcgagacat   1260
   tgccgcatca aggatttgca taccgtacac atgtttctcg acgcgtcatc tgtggaaatc   1320
   tttatcaata acggagaaga ggtctttagt gcaagatatt ttcctttccc gggaaatcat   1380
   gaagtaacag ccagtgcgac cgggaaatct gaaatgaatg tcggaatttg gacacttatg   1440
   tag                                                                 1443
<210> 11
   <211> 30
   <212> DNA
   <213> primer
<400> 11
   ggaattcatg ctcgttttag ctagaattgg 30
<210> 12
   <211> 35
   <212> DNA
   <213> primer
<400> 12
   tccgagctct tactattctt ttgcgttagc tcttg 35
<210> 13
   <211> 52
   <212> DNA
   <213> primer
<400> 13
   acctgcgagc tctttcacac aggaaacaat tttcatgcgg tcgtttttac cg 52
<210> 14
   <211> 75
   <212> DNA
   <213> primer
<400> 14
   caaattttgt ttgtcatatg catgaaatct gtttcctgtg tgaaattact atttatattc 60
   aatttctttc ggata 75
<210> 15
   <211> 75
   <212> DNA
   <213> primer
<400> 15
   tatccgaaag aaattgaata taaatagtaa tttcacacag gaaacagatt tcatgcatat 60
   gacaaacaaa atttg 75
<210> 16
   <211> 38
   <212> DNA
   <213> primer
<400> 16
   acctgcgggt accctattag tttgctaaaa attccgct 38
<210> 17
   <211> 20
   <212> DNA
   <213> primer
<400> 17
   ggaaacagac catggaattc 20
<210> 18
   <211> 24
   <212> DNA
   <213> primer
<400> 18
   ccgcaaaaga tttattcgaa gaag 24
<210> 19
   <211> 22
   <212> DNA
   <213> primer
<400> 19
   cctggttata tgatacttta gg 22
<210> 20
   <211> 24
   <212> DNA
   <213> primer
<400> 20
   tagtgctggg cgcaagagct aacg 24
<210> 21
   <211> 20
   <212> DNA
   <213> primer
<400> 21
   accagtgggc gataaaatcg 20
<210> 22
   <211> 20
   <212> DNA
   <213> primer
<400> 22
   tgatcaaggt ttcgatttct 20
<210> 23
   <211> 20
   <212> DNA
   <213> primer
<400> 23
   ttttcctgaa tgacggcgaa 20
<210> 24
   <211> 20
   <212> DNA
   <213> primer
<400> 24
   cgatctgccg caatttcaag 20
<210> 25
   <211> 32
   <212> DNA
   <213> primer
<400> 25
   atatctgcag ccggcattaa atattagtca ac 32
<210> 26
   <211> 30
   <212> DNA
   <213> primer
<400> 26
   cgttctaacg gaggttgaaa actgcccttt 30
<210> 27
   <211> 31
   <212> DNA
   <213> primer
<400> 27
   gtctccctat cacgccgtta ttttcattat t 31
<210> 28
   <211> 30
   <212> DNA
   <213> primer
<400> 28
   attagtcgac accatcccca cggaatacat 30
<210> 29
   <211> 30
   <212> DNA
   <213> primer
<400> 29
   tttcaacctc cgttagaacg cggctacaat 30
<210> 30
   <211> 30
   <212> DNA
   <213> primer
<400> 30
   taacggcgtg atagggagac cggcagatcc 30
<210> 31
   <211> 31
   <212> DNA
   <213> primer
<400> 31
   atacactgca gttatgcaat ttatcgcacc c 31
<210> 32
   <211> 33
   <212> DNA
   <213> primer
<400> 32
   aatctgctct gatgcggtcg tgaaatgctt cca 33
<210> 33
   <211> 32
   <212> DNA
   <213> primer
<400> 33
   cacagaatca ggacaaatgg cattcaatgc tg 32
<210> 34
   <211> 29
   <212> DNA
   <213> primer
<400> 34
   atactgtcga ctcaatggca tatgcagcg 29
<210> 35
   <211> 40
   <212> DNA
   <213> primer
<400> 35
   aagcatttca cgaccgcatc agagcagatt gtactgagag 40
<210> 36
   <211> 41
   <212> DNA
   <213> primer
<400> 36
   ttgaatgcca tttgtcctga ttctgtggat aaccgtatta c 41
<210> 37
   <211> 20
   <212> DNA
   <213> primer
<400> 37
   cggggcgaaa gtgattgaga 20
<210> 38
   <211> 20
   <212> DNA
   <213> primer
<400> 38
   aattgccgcc tgggtattgg 20
<210> 39
   <211> 22
   <212> DNA
   <213> primer
<400> 39
   acctttacta ccgcactgct gg 22
<210> 40
   <211> 22
   <212> DNA
   <213> primer
<400> 40
   gcgggagtca gtgaacaggt ac 22
<210> 41
   <211> 25
   <212> DNA
   <213> primer
<400> 41
   gatcttgagt ccgtaaaaca ggctt 25
<210> 42
   <211> 22
   <212> DNA
   <213> primer
<400> 42
   ttccgctcaa gccattgtag tg 22
<210> 43
   <211> 33
   <212> DNA
   <213> primer
<400> 43
   actgagccat ggcgaaaatc aataaagtag atc 33
<210> 44
   <211> 34
   <212> DNA
   <213> primer
<400> 44
   tgatccgagc tcctattatt ccagtgttcc cgcc 34
<210> 45
   <211> 32
   <212> DNA
   <213> primer
<400> 45
   actccggaat tcatgacgca atctcgattg ca 32
<210> 46
   <211> 35
   <212> DNA
   <213> primer
<400> 46
   acctgcgagc tcccgttgtt ccacctgata ttatg 35
<210> 47
   <211> 34
   <212> DNA
   <213> primer
<400> 47
   gcatagaatt catgacagca catgaccagg agct 34
<210> 48
   <211> 39
   <212> DNA
   <213> primer
<400> 48
   gcatagagct cctacataag tgtccaaatt ccgacattc 39
<210> 49
   <211> 20
   <212> DNA
   <213> primer
<400> 49
   cccgttctgg ataatgtttt 20
<210> 50
   <211> 20
   <212> DNA
   <213> primer
<400> 50
   aaagtcacgg ttgttattcc 20
<210> 51
   <211> 20
   <212> DNA
   <213> primer
<400> 51
   catttaatgc cgctcatatt 20
<210> 52
   <211> 20
   <212> DNA
   <213> primer
<400> 52
   accgctcaat tattgagatt 20

## Description

### TECHNICAL FIELD

The present invention relates to a recombinant microorganism capable of metabolizing sucrose, and more particularly to a recombinant microorganism capable of metabolizing sucrose in which a gene encoding sucrose phosphotransferase and/or a gene encoding sucrose-6-phosphate hydrolase is introduced or to a recombinant microorganism capable of metabolizing sucrose in which a gene encoding β-fructofuranosidase is introduced, where in the pts G gene has SEQ ID no.2 and the sucrose-6-phosphate hydrolase is a sacC gene.

### BACKGROUND ART

For the sustainable development of mankind, studies for the development of the industrial biotechnology for the production of useful compounds from renewable bio-resources are being actively conducted along with a great interest therein. The production of chemical substances by microbial fermentation has been performed to date using glucose as a main raw material. However, the production of chemical products by microbial fermentation is difficult to commercialize, because glucose is expensive and thus the price of chemical compounds produced by the microbial fermentation is higher than that of chemical compounds produced by chemical synthetic methods that use crude oil as a main raw material. Thus, to develop as an alternative to the method that uses expensive glucose as a carbon source, studies on the discovery of a variety of inexpensive carbon sources which can be easily obtained from abundant bioresources are being actively conducted. For example, studies on the production of various primary metabolites using relatively inexpensive raw materials, such as lignocellulosic hydrolysates, glycerol, whey, corn steep liquors or the like, have been conducted by many researchers including the present inventors (Samuelov et al., Appl. Environ. Microbiol., 65:2260, 1999; Lee et al., Appl. Microbiol. Biotechnol., 54:23, 2000; Lee et al., Biotechnol. Bioeng., 72:41, 2001; Lee et al., Biotechnol. Lett., 25:111, 2003; Lee et al., Bioproc. Biosystems Eng., 26:63, 2003). However, according to the study results reported to date, when the raw materials were used as carbon sources instead of glucose, the productivity or production yield of desired metabolites was significantly lower than when glucose was used as a carbon source. Thus, research and development are urgently required to overcome this drawback.

Sucrose (commonly known as sugar) is a disaccharide consisting of glucose and fructose, and it is a carbon source that is very abundant in nature and is produced from all plants having photosynthesis ability. Particularly, sugarcane and sugar beet contain large amounts of sucrose, and more than 60% of the world's sucrose is currently being produced from sugarcane. Particularly, sucrose is produced at a very low cost, because it can be industrially produced through a simple process of evaporating/concentrating extracts obtained by mechanical pressing of sugarcanes. Koutinas *et al.* calculated the prices of various raw materials usable for the microbial production of chemical substances on the basis of the glucose contents in the year 2004 and, as a result, the price of sucrose based on 1 kg of the glucose content was 26.1 cents which is a very low price corresponding to 77% of the wheat price, 50% of the molasses price and 28.9% of the sucrose price (Koutinas et al., Ind. Crops and Products, 20:75, 2004).

A report on the International Sugar Agreement (ISA) daily price indicates that the price of sucrose is on a steady downward trend after peaking in 1994-1995 due to surplus supply and that the downward trend is expected to continue. Accordingly, sucrose is receiving attention as the most potent carbon source which will substitute for expensive glucose that is currently being used to produce various chemical compounds through microbial fermentation. Particularly, it is well known to those skilled in the art to it is very difficult to reduce the production cost of glucose to the level of sucrose, because glucose that is produced mainly from corn starch is produced through very complicated processes including extraction of starch from corn, thermal/chemical pretreatment of starch, conversion of starch to glucose by enzymatic reactions, and purification of glucose, and because the price of corn is continuously increasing. For these reasons, corn-based bioethanol production in the USA is gradually decreasing (Mae-Wan Ho, Science in Society, 33:40, 2007), but sugarcane (i.e., sucrose)-based bioethanol production in Brazil is rapidly growing.

To date, studies on the production of useful compounds using sucrose as a carbon source have been conducted with respect to the production of biodegradable polymers (Lee et al., Biotechnol. Lett., 15:971, 1993; Lee et al., Biotechnol. Techniques, 1:59, 1997), citric acid (Forster et al., App. Microbiol. Biotechnol., 75:1409, 2007), acetone, butanol, ethanol and isopropanol (George et al., Appl. Environ. Microbiol., 45:1160, 1983; Durre, Appl. Microbiol. Biotechnol., 49:639, 1998), itaconic acid (Kautola et al., Biotechnol. Lett., 11:313, 1989), xanthan gum (Letisse et al., Appl. Microbiol. Biotechnol., 55:417, 2001), etc., by high-concentration cell culture. Particularly, the report (2006) of the International Energy Agency (IEA) Bioenergy Task 40, which analyzes international bioenergy and biofuel trade evaluated that bioethanol production from sugarcane (including sucrose) in Brazil is an excellent model of environmentally friendly, sustainable biofuel production.

Sucrose as a raw material for producing chemical compounds through microbial fermentation is inexpensive and can function to protect the cell membrane from an external environment containing large amounts of desired metabolites, thus producing high-concentrations of desired metabolites. Kilimann *et al.* exposed microorganisms to a medium containing sucrose and a medium containing no sucrose at lethal temperatures and then examined the viability thereof and the secondary structures of the proteins (Biochimica et Biophysica Acta, 1764, 2006).

The study results revealed that the secondary structures of proteins in the cells of the microorganisms exposed to the medium containing sucrose were very well conserved, but the structures of proteins in the cells of the microorganisms exposed to the medium containing sucrose were highly modified. Particularly, the viability of the microorganisms exposed to the medium containing sucrose was significantly higher than that of the microorganisms exposed to the medium containing no sucrose. This directly demonstrates the function of sucrose to protect microorganisms from a harmful external environment.

Even though sucrose is an excellent raw material having the above-described advantages, including low price and a function to protect microorganisms from an external environment, an example showing the successful production of desired chemical compounds using sucrose as a carbon source and the actual commercial application thereof has not yet been reported. This is because a large number of microorganisms do not have a complete mechanism of transporting sucrose into cells, degrading the transported sucrose and linking the degraded products to glycolysis, and thus cannot use sucrose as a carbon source. Even in the case of microorganisms having a mechanism capable of using sucrose, they cannot efficiently produce desired metabolites, because the rate of ingestion and degradation of sucrose as a carbon source is very low.

Jahreis et al., 2002 discloses recombinant vectors suitable for use in microorganisms carrying the genes *cscA* and cscB from *E*. *coli* encoding, respectively, a sucrose hydrolase and a sucrose permease. WO2007/04126 also discloses recombinant organisms able to metabolise sucrose and carrying *cscA* and cscb from *E*. *coli.*

In order for the production of various chemical compounds through microbial fermentation to be performed in an industrially economic manner, the use of an inexpensive raw material such as sucrose as described above is required, and furthermore, the identification of an enzyme capable of efficiently ingesting and degrading sucrose as a carbon source at a high rate and the research and development enabling the use of the enzyme are necessarily required. Particularly, in view of the fact that the price of raw materials for producing chemical compounds through microbial fermentation account for about 50% of the price of final products, the identification of an enzyme enabling efficient use of sucrose as an inexpensive raw material and the development of the application of the enzyme are urgently required.

It has been reported that sucrose can be used for producing various bioproducts, including biodegradable polymers, citric acid, itaconic acid, acetone, butanol, ethanol, isopropanol and xanthan gum, by high-concentration cell culture. However, examples showing the successful production of desired chemical compounds through microbial fermentation and the actual commercial application thereof have been rarely reported.

Thus, in order for the production of various chemical compounds through microbial fermentation as an environmentally friendly technology to be successfully applied in the industry, the development of microorganisms capable of effectively ingesting and degrading an inexpensive and abundant carbon source such as sucrose is required.

### SUMMARY OF INVENTION

In its broadest sense, the invention concerns subject-matter as defined in the appended claims.

It is, therefore, an object of the present invention to provide novel sucrose metabolism-related genes enabling sucrose to be used as a carbon source, and enzymes which are encoded by the genes.

Another object of the present invention is to provide a recombinant microorganism capable of metabolizing sucrose in which the sucrose metabolism-related gene is introduced, and a method for producing metabolites, biodegradable polymer or recombinant proteins using the recombinant microorganism.

In order to achieve the above objects, the present invention provides a sucrose phosphotransferase having an amino acid sequence of SEQ ID NO: 1; a gene (*ptsG*) encoding said sucrose phosphotransferase; and a recombinant vector containing said gene *(ptsG)* and a gene (*sacC*) encoding a sucrose-6-phosphate hydrolase.

The present invention also provides a recombinant microorganism capable of metabolizing sucrose in which said gene is introduced into a host cell selected form the group consisting of bacteria, yeast and fungi; and a method for producing metabolites, biodegradable polymers or recombinant proteins, the method comprises culturing said recombinant microorganism in a medium containing sucrose as a carbon source.

The present disclosure also provides a β-fructofuranosidase having an activity to hydrolyze β-D-fructofuranoside bond to liberate fructose; and a gene encoding said β-fructofuranosidase.

The present invention also provides a recombinant microorganism capable of metabolizing sucrose in which said gene is introduced into a host cell selected form the group consisting of bacteria, yeast and fungi; and a method for producing metabolites, biodegradable polymers or recombinant proteins, the method comprises culturing said recombinant microorganism in a medium containing sucrose as a carbon source.

Other features and aspects of the present invention will be more apparent from the following detailed description and the appended claims.

### BRIEF DESCRIPTION OF DRAWINGS

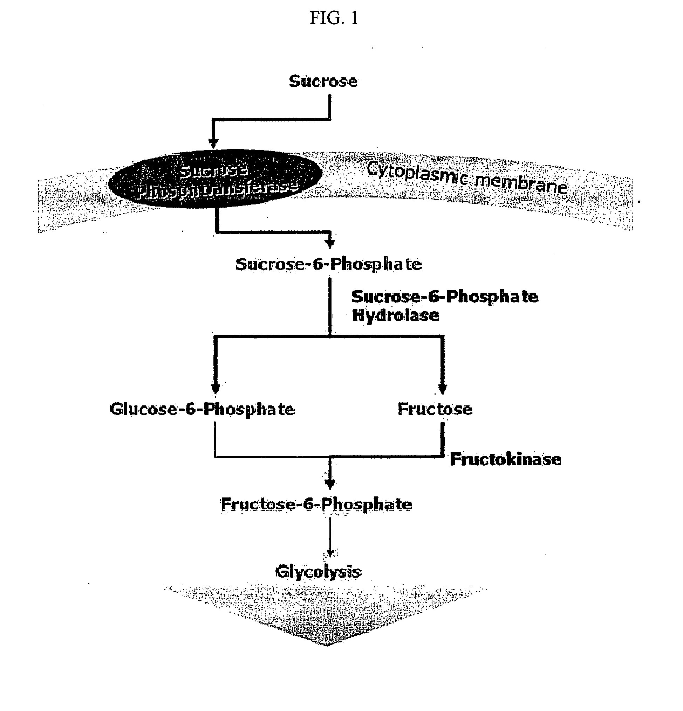

FIG. 1 is a schematic diagram showing a pathway through which sucrose is ingested and degraded, and the degraded products are metabolized through glycolysis, when novel metabolism-related enzymes (sucrose phosphotransferase, sucrose-6-phosphate hydrolase, and fructokinase) derived from *M. succiniciproducens* MBEL55E, which enable the metabolism of sucrose, are introduced into a microorganism incapable of metabolizing sucrose. Thick arrows (→): indicates metabolic pathways in which the introduced novel sucrose metabolism-related enzymes derived from *M. succiniciproducens* MBEL55E are involved; and thin arrows (→): indicate the original metabolic pathways of the recombinant microorganism.
FIG. 2 is a schematic diagram showing four possible pathways how a novel β-fructofuranosidase derived from *M. succiniciproducens* MBEL55E, which enables the metabolism of sucrose, can be involved in sucrose metabolism, when the enzyme is introduced into a microorganism incapable of metabolizing sucrose. Thick arrows (→): four possible pathways in which the introduced novel β-fructofuranosidase derived from *M succiniciproducens* MBEL55E will be involved; and thin arrows (→): indicate the original metabolic pathways of the recombinant microorganism.
FIG. 3 is a map of recombinant vector pMSscrIIA containing genes (*ptsG*, *sacC* and *rbsK*) encoding sucrose phosphotransferase, sucrose-6-phosphate hydrolase and fructokinase.
FIG. 4 is a graphic diagram showing the growth of parent strain MBEL55E, a recombinant MptsG strain and a recombinant MsacC strain in sucrose media (●: MBEL55E; ▲ : MptsG; and Δ : MsacC).
FIG. 5 is a cleavage map of recombinant vector pTac15K.
FIG. 6 is a cleavage map of recombinant vector pTac15KsacC containing a gene encoding sucrose-6-phosphate hydrolase.
FIG. 7 is a graphic diagram showing the growth of recombinant *E. coli* W3110 pTac15K in a M9 medium containing sucrose (solid line including ● : sucrose concentration; and solid line including ◆ : OD₆₀₀).
FIG. 8 is a graphic diagram showing the growth of the inventive recombinant *E*. *coli* W3110 pTac15KsacC, having the ability to metabolize sucrose, in a M9 medium containing sucrose (solid line including ● : sucrose concentration; solid line including ◆ : OD₆₀₀; dot line including ● : glucose concentration; solid line including ○ : fructose concentration; and dot line including ▼ : acetic acid concentration).
FIG. 9 is a graphic diagram showing the growth of *E*. *coli* W3110 pTac15KEWcscA and *E*. *coli* W3110 pTac15KBSsacA in M9 media containing sucrose (solid line including ● : *E. coli* W3110 pTac15KEWcscA; and solid line including ▲ : *E. coli* W3110 pTac15KBSsacA).

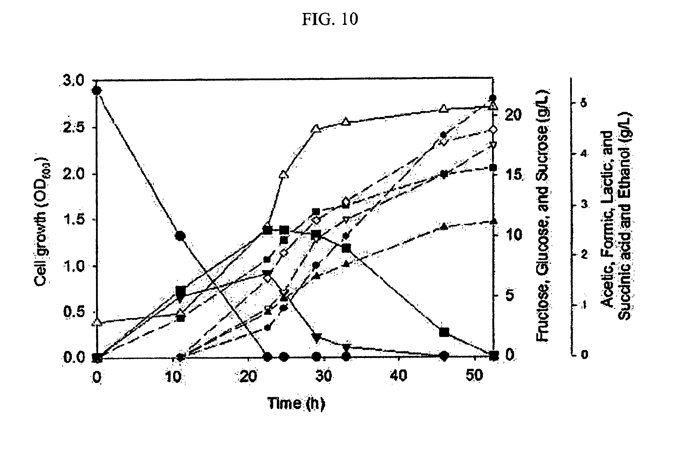

FIG. 10 is a graphic diagram showing the growth and metabolite production of the inventive *E*. *coli* W3110 pTac15KsacC capable of metabolizing sucrose, fermented in anaerobic conditions (solid line including ● : sucrose concentration; solid line including Δ : OD₆₀₀; solid line including ▼ : glucose concentration; solid line including ■ : fructose concentration; dot line including ● : succinic acid concentration; dot line including ∇ : lactic acid concentration; dot line including ■ : formic acid concentration; dot line including ◊ : acetic acid concentration; and dot line including ▲ : ethanol concentration).

### DETAILED DESCRIPTION OF THE INVENTION, AND PREFERRED EMBODIMENTS

The present invention aims at the production of bioproducts using sucrose (commonly known as sugar) as an inexpensive and abundant carbon source, particularly the discovery of either a microorganism capable of using sucrose as a carbon source or enzymes enabling the use of a microorganism incapable of using sucrose, and aims to use the enzymes to produce bioproducts.

Sucrose which is a disaccharide consisting of glucose and fructose is a globally abundant carbon source. It is produced from most plants having photosynthesis ability, and particularly, sugarcane and sugar beet which are tropical crops and subtropical crops contain large amounts of sucrose. Sucrose can be industrially produced through a simple process of evaporating and concentrating an extract obtained by mechanical pressing of sugarcane, and more than 60% of the world's sucrose is currently being produced from sugarcane. According to the paper (published in 2004) of Koutinas *et al.* who calculated the prices of various raw materials usable in microbial fermentation on the basis of the glucose contents, the price of sucrose based on 1 kg of the glucose content is 26.1 cents which is 1/4 of the glucose price and 1/2 to 2/3 of the wheat and molasses prices (Koutinas et al., Ind. Crops and Products, 20:75, 2004). Furthermore, with respect to the change in the sucrose price published in the International Sugar Organization for recent 15 years, the sucrose price declined sharply to 6.27 cents/lb in 1999 after peaking to 13.28 cents/lb in 1995, increased again to 14.20 cents/lb on March, 2008 and was maintained at a level of 12.44 cents/lb on 7 May, 2008, and the sucrose price is expected to be on a downward trend due to surplus supply. In addition to this advantage in terms of cost, sucrose is advantageous in that it can be relatively stably supplied even in food crisis circumstances such as recent grain crisis, because it is not obtained from grain.

To date, it has been reported that sucrose can be used for producing various bioproducts, including biodegradable polymers, citric acid, itaconic acid, acetone, butanol, ethanol, isopropanol and xanthan gum (Lee et al., Biotechnol. Lett., 15:971, 1993; Lee et al., Biotechnol. Techniques, 1:59, 1997; Forster et al., App. Microbiol. Biotechnol., 75:1409, 2007; George et al., Appl. Environ. Microbiol., 45:1160, 1983; Durre, Appl. Microbiol. Biotechnol., 49:639, 1998; Kautola et al., Biotechnol. Lett., 11:313, 1989; Letisse et al., Appl. Microbiol. Biotechnol., 55:417, 2001), by high-concentration cell culture. This suggests that an improvement in the utility of sucrose can have a direct influence on the effective production of bioproducts.

However, despite several advantages of sucrose, including advantages as a raw materials, a function to protect proteins from modification, and a function to protect cells from external environments (Kilimann et al., Biochimica et Biophysica Acta, 1764, 2006), examples showing the successful production of desired chemical compounds through microbial fermentation using sucrose as a carbon source and the actual commercial application thereof have been rarely reported. One reason therefor is that a large number of microorganisms cannot effectively produce desired bioproducts, because the microorganisms do not have a mechanism capable of metabolizing sucrose or have a slow metabolic rate, even if they have the mechanism. Thus, in order for the production of various chemical compounds through microbial fermentation as an environmentally friendly technology to be successfully applied in the industry, the development of microorganisms capable of effectively ingesting and degrading an inexpensive and abundant carbon source such as sucrose is required. For this purpose, the identification of an enzyme capable of degrading and metabolizing at high rate and the utilization thereof must be performed.

To date, an enzyme group enabling the use of sucrose has been developed by several researchers. Typical examples include the technology of Ajinomoto Co. in which *E. coli*-derived PTS (phosphoenol pyruvate-dependent phosphotransferase system) and a sucrose transport system of non-PTS are introduced and used to produce amino acids. This technology has a characterized in that a whole gene group associated with PTS or non-PTS is introduced, thus completing an invention.

However, in the present invention, based on the genetic information of *Mannheimia succiniciproducens* MBEL55E (KCTC 0769BP), novel genes (*ptsG, sacC* and *rbsK*) encoding enzymes [sucrose phosphotransferase (PtsG, MS0784), sucrose-6-phosphate hydrolase (SacC, MS0909) and fructokinase (RbsK, MS1233)] which are involved in transporting sucrose into cells, degrading the transported sucrose and linking the degraded products to glycolysis were found, and the sequences and functions thereof were identified.

Accordingly, in one aspect, the present invention relates to sucrose phosphotransferase having an amino acid sequence of SEQ ID NO: 1, sucrose-6-phosphate hydrolase having an amino acid sequence of SEQ ID NO: 3, and fructokinase having an amino acid sequence of SEQ ID NO: 5, which are enzymes that are involved in transporting sucrose into cells, degrading the transported sucrose and linking the degraded products to glycolysis. The present invention also relates to a gene *(ptsG)* encoding said sucrose phosphotransferase, a gene (*sacC*) encoding said sucrose-6-phosphate hydrolase, and a gene (*rbsK*) encoding said fructokinase.

In the present invention, the sucrose phosphotransferse (PtsG) functions to transport sucrose into cells while converting sucrose into sucrose-6-phosphate, the sucrose-6-phosphate hydrolase (SacC) has an activity to convert sucrose-6-phosphate to glucose-6-phosphate and fructose, and the fructokinase (RbsK) has an activity to convert fructose to fructose-6-phosphate.

In the present invention, the *ptsG* is represented by a base sequence of SEQ ID NO: 2, the *sacC* is represented by a base sequence of SEQ ID NO: 4, and *rbsK* is represented by a base sequence of SEQ ID NO: 6.

Specifically, in the present invention, a recombinant vector containing the *ptsG* and *sacC* genes was constructed, and then introduced into *E. coli* incapable of using sucrose as a carbon source, and the constructed recombinant *E. coli* was cultured in a medium containing sucrose as a single carbon source. As a result, it was found that the recombinant E. *coli* had the ability to metabolite sucrose.

Accordingly, as shown in FIG. 1, it can be inferred that sucrose is transported into cells by the sucrose phosphotransferase (PtsG) while being converted to sucrose-6-phosphate, the sucrose-6-phosphate transported into cells is converted to glucose-6-phosphate and fructose by the sucrose-6-phosphate hydrolase (SacC), the fructose is converted to frustose-6-phosphate by the fructokinase (RbsK), and the degraded product are linked to glycolysis.

Meanwhile, the present inventors have demonstrated that microorganisms which have not been capable of metabolizing sucrose can metabolize sucrose by the introduction of sucrose-6-phosphate hydrolase (SacC, MS0909) that is β-fructofuranosidase derived from *Mannheimia succiniciproducens* MBEL55E (KCTC 0769BP). Other genes (*cscA* and *sacA*) encoding fructofuranosidase, represent examples of producing various metabolites using the microbial strain. In other words, based on the genetic information of *Mannheimia succiniciproducens* MBEL55E (KCTC 0769BP), *E. coli* W and *Bacillus subtilis,* novel genes (*sacC, cscA,* and *sacA*) encoding β-fructofuranosidase that is an enzyme involved in degrading sucrose and linking the degraded products to glycolysis were discovered, and the sequences and functions thereof were identified. EC number (Enzyme Commission number) provided by the Nomenclature Committee of the International Union of Biochemistry and Molecular Biology is a well-known numerical classification scheme for enzymes, based on the chemical reactions they catalyze.

The official name for sucrose-6-phosphate hydrolase is β-fructofuranosidase, (EC 3.2.1.26)) and has other names including β-D-fructofuranoside fructohydrolase, invertase, saccharase, glucosucrase, β-h-fructosidase, β-fructosidase, invertin, sucrase, maxinvert L 1000, fructosylinvertase, alkaline invertase, acid invertase . Namely, sucrose-6-phosphate hydrolase and sucrase all have the official name β-fructofuranosidase (EC 3.2.1.26).

Such β-fructofuranosidase catalyzes the hydrolysis of terminal non-reducing β-D-fructofuranoside residues in β-D-fructofuranoside. It has the official name "sucrase or invertase" when it catalyzes the hydrolysis of sucrose, and has the official name "sucrose-6-phosphate hydrolase" when it catalyzes the hydrolysis of sucrose-6-phosphate.

In Examples herein, in addition to demonstrating the sucrose-metabolizing ability caused by the introduction of sucrose-6-phosphate hydrolase (EC 3.2.1.26, SacC) derived from *Mannheimia,* that is, β-fructofuranosidase, an attempt was made to demonstrate the introduction of general β-fructofuranosidase into microorganisms imparts the sucrose-metabolizing ability to the microorganisms. As a result, when each of *E*. *coli* W-derived invertase (EC 3.2.1.26, CscA) and *Bacillus subtilis-derived* β-fructofuranosidase was introduced into microorganisms incapable of metabolizing sucrose, it was observed that the microorganisms introduced with β-fructofuranosidase (EC 3.2.1.26) grew by metabolizing sucrose.

Accordingly, the disclosure relates to β-fructofuranosidase having activities to hydrolyze β-D-fructofuranoside bond to liberate fructose, including an activity to hydrolyze sucrose to glucose and fructose and an activity to hydrolyze sucrose-6-phosphate to glucose-6-phosphate and fructose. Also, the β-fructofuranosidase may have an amino acid sequence selected from the group consisting of amino acid sequences of SEQ ID NO: 3, SEQ ID NO: 7 and SEQ ID NO: 9.

Specifically, in the present invention, sucrose-6-phospahte hydrolase (Sacc) has an activity to convert sucrose-6-phosphate to glucose-6-phosphate and fructose or an activity to convert sucrose to glucose and fructose. Also, β-fructofuranosidase derived from *Mannheimia* was used having an amino acid sequence of SEQ ID NO: 3, and a gene (*sacC*) encoding the same is represented by a base sequence of SEQ ID NO: 4.

In the present disclosure, invertase, sucrase and sucrose hydrolase (CscA) that are examples of the β-fructofuranosidase are enzymes which are involved in degrading sucrose and linking the degraded products to glycolysis, and these enzymes have an activity to convert sucrose-6-phosphate to glucose-6-phosphate and fructose or an activity to convert sucrose to glucose and fructose. Furthermore, in Examples, the β-fructofuranosidase derived from *E*. *coli* W was illustrated, and may have an amino acid sequence of SEQ ID NO: 7, and a gene (*cscA*) encoding the same is preferably represented by a base sequence of SEQ ID NO: 8.

Sucrose-6-phosphate hydrolase (SacA) that is one example of the β-fructofuranosidase is an enzyme which is involved in degrading sucrose and linking the degraded products to glycolysis and has an activity to convert sucrose-6-phosphate to glucose-6-phosphate and fructose and an activity to convert sucrose to glucose and fructose. In Examples the β-fructofuranosidase derived from *Bacillus subtilis* was illustrated and may have an amino acid sequence of SEQ ID NO: 9, and a gene (*sacA*) encoding the same is preferably represented by a base sequence of SEQ ID NO: 10 (*sacA,* BSU38040, sucrose-6-phosphate hydrolase).

Moreover, when the amino acid sequence of the *Mannheimia-derived* β-fructofuranosidase encoded by the *sacC* gene were compared with the amino acid sequence of enzymes searched through protein BLAST , invertase encoded by *E*. *coli* W-derived *cscA* has an amino acid sequence identity of 28%, and sucrose-6-phosphate hydrolase β-fructofuranosidase) encoded by *Bacillus subtilis-derived sacA* has an amino acid sequence identity of 35%. Also, the β-fructofuranosidases derived from the two strains all have the conserved domain β- fructosidase (COG1621) designated as "SacC". This indicates that, when any enzyme which has an amino acid sequence somewhat different from the *Mannheimia*-derived β-fructofuranosidase encoded by the *sacC* gene, but contains the conserved domain β-fructosidase (COG1621) designated as "SacC", is introduced into microorganisms as described below, the microorganisms can grow by metabolizing sucrose. Therefore, although the β-fructofuranosidase has been illustrated by an amino acid sequence of SEQ ID NO: 3, 7 or 9, the scope of the present invention is not limited thereto. Namely, all β-fructofuranosidases can be included in the scope of the present invention, as long as they have an activity to hydrolyze the β-D-fructofuranoside bond to liberate fructose. For example, amino acid sequences having an amino acid sequence identity of at least 70%, 80% or 90% to the amino acid sequence of SEQ ID NO: 3, 7 or 9 may also be included in the scope of the present invention.

Likewise, the gene encoding gene the β-fructofuranosidase may have, for example, a base sequence of SEQ ID NO: 4, 8 or 10. DNA comprising a mutation (substitution, deletion, insertion or addition) in one or more bases in these sequences and having a sequence identity of at least 70% or 80%, preferably 90%, and more preferably 95% compared to the base sequence according to the present invention.

The term "sequence identity" as used herein refers to sequence similarity between two polynucleotides or two nucleic acid molecules. The sequence identity can be determined by comparing two optimally aligned sequences over a comparison window, where the fragment of the polynucleotide or amino acid sequence in the comparison window may comprise additions or deletions (e.g., gaps or overhangs) as compared to the reference sequence (e.g., a consensus sequence) for optimal alignment of the two sequences. The percentage of sequence identity can be calculated by determining the number of positions at which the identical nucleic acid base or amino acid residue occurs in both sequences to yield the number of matched positions, dividing the number of matched positions by the total number of positions in the window of comparison and multiplying the result by 100 to yield the percentage of sequence identity. The sequence identity between nucleic acid or amino acid sequences can be measured using sequence analysis software, for example, BLASTN or BLASTP.

In the present invention, as described above, sucrose which is a disaccharide consisting of D-glucose and D-fructose is known to function to prevent modification of proteins in cells, stabilize proteins in cells and minimize the lysis of cells caused by the change in external environments. Thus, sucrose is highly useful in the production of high concentrations of basic chemical compounds or in high-concentration cell culture.

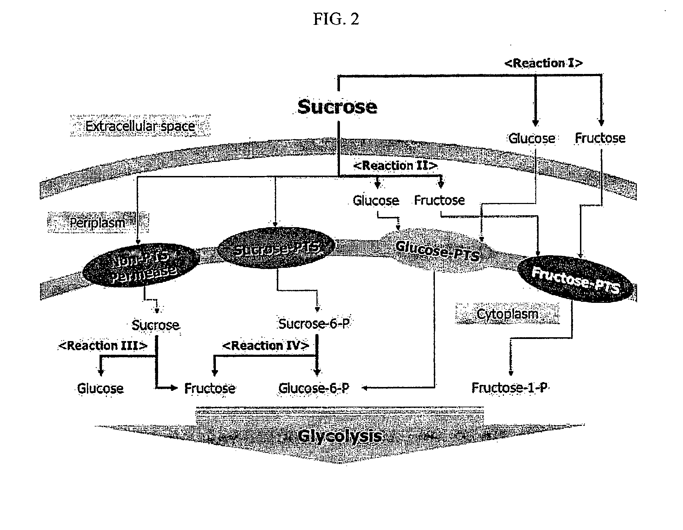

As shown in FIG. 2, when sucrose-6-phosphate hydrolase which is a novel β-fructofuranosidase derived from *M succiniciproducens* MBEL55E is introduced into microorganisms incapable of metabolizing sucrose, the introduced enzyme is believed to metabolize sucrose or sucrose-6-phosphate through four possible pathways.

The first possible pathway (Reaction I) is the case in which the sucrose-6-phosphate hydrolase degrades sucrose to glucose and fructose in the extracellular space of cells, and then the degraded products are introduced into cells by enzymes which are involved in transport, such as respective phosphotransferase.

The second possible pathway (Reaction II) is the case in which sucrose-6-phosphate hydrolase degrades sucrose to glucose and fructose in the periplasm, and then the degraded products are introduced into cells by enzymes which are involved in transport, such as respective phosphotransferase.

The third possible pathway (Reaction III) is the case in which sucrose is introduced into cells by permease enzyme other than the phosphotransferase family, and then degraded by the introduced sucrose-6-phosphate hydrolase into glucose and fructose.

The fourth possible pathway (Reaction IV) is the case in which sucrose is converted to sucrose-6-phosphate by phosphate transferase while being introduced into cells, and then converted into fructose and glucose-6-phosphate by the introduced sucrose-6-phosphate hydrolase.

Also, the *sacC* gene is derived from *M succiniciproducens* MBEL55E (KCTC 0769BP) which belongs to the family *Pasteurellaceae* together with *Actinobacillus succinogenes* 130Z (ATCC 55618). Particularly, said *M succiniciproducens* MBEL55E (KCTC 0769BP) and *A. succinogenes* 130Z (ATCC 55618) have similar genomic sequences and cell physiologies. The genomic sequences of the two microbial strains are known to be the most similar to each other among all genomic sequences decoded to date (McKinlay et al., Appl. Microbiol. Biotechnol., 76:727, 2007). Also, It is known that the *sacC* gene derived from *A. succinogenes* 130Z has a very high homology with one derived from *M succiniciproducens* MBEL55E and is the most similar thereto. Thus, it is obvious to those skilled in the art that a *M succiniciproducens* MBEL55E-derived sucrose-6-phosphate hydrolase (SacC, MS0909) enzyme and a gene encoding the same, and an *A. succinogenes* 130Z-derived sucrose-6-phosphate hydrolase (Asuc_1829) enzyme and a gene encoding the same, can likewise be applied.

Accordingly, in another still aspect, the present invention relates to a recombinant microorganism capable of metabolizing sucrose in which a gene encoding sucrose phosphotransferase and/or sucrose-6-phosphate hydrolase is introduced, and a method for producing metabolites, biodegradable polymers or recombinant proteins, which comprises culturing said recombinant microorganism in a medium containing sucrose as a carbon source.

In the present invention, the recombinant vector which is a vector capable of expressing a protein in a suitable host cell refers to a DNA construct containing a DNA sequence operably linked to control sequences capable of controlling the expression of a protein together with other sequences which facilitate the manipulation of genes, optimize the expression of proteins or are required for the replication of the vector. The control sequences may include a promoter for regulating transcription, an operator optionally added for regulating transcription, a suitable mRNA ribosome binding site and/or sequences controlling the termination of transcription/translation.

For example, the recombinant vector may be a recombinant vector such as pTrc99A used in Examples of the present invention, but in addition to this vector, other known vectors may be used in the present invention. Also, an expression cassette containing the *sacC* gene may be inserted not only into expression vectors such as pKK223-3, pTac99A, pET series or pMAL series, but also into a cloning vectors such as pACYC, pBluescript SK-, pBR322, pGEM series or pMB1, and such expression vectors may be used in the present invention. In addition, it is possible to use recombinant vectors known in the art to which the present invention pertains (Sambrook J & Russell D, Molecular Cloning: a laboratory manual, 3rd ed., Cold Spring Harbor Lab (CSHL) Press, New York, 2001). Furthermore, vectors containing, in addition to an ampicillin-resistant gene, several other resistant genes known in the art, may also be used in the present invention.

The above-described genes are derived from *M. succiniciproducens* MBEL55E (KCTC 0769BP) which belongs to the family *Pasteurellaceae* together with *Actinobacillus succinogenes* 130Z (ATCC 55618). Particularly, the two strains, *M. succiniciproducens* MBEL55E (KCTC 0769BP) and *A. succinogenes* 130Z (ATCC 55618), have very similar genome sequences and cell physiologies. According to the report (2007) of McKinlay *et al.,* it is known that the genome sequences of the two microbial strains are the most similar to each other among all genome sequences decoded to date *(*Appl. Microbiol. Biotechnol., 76:727, 2007). Thus, it is obvious to those skilled in the art that the above genes are also applied to genes derived from *A. succinogenes* 130Z (ATCC 55618) together with *M. succiniciproducens* MBEL55E (KCTC 0769BP).

In Examples of the present invention, a microorganism incapable of using sucrose as a carbon source was transformed using a recombinant vector containing the *ptsG, sacC* and *rbsK* genes, thus constructing a recombinant microorganism having the ability to metabolize sucrose. However, a genome-engineered recombinant microorganism may also be constructed by inserting the above genes into the chromosome of a microorganism incapable of using sucrose as a carbon source according to a method well known in the art.

In the present invention, a recombinant vector containing β-fructofuranosidase-encoding genes including the *sacC* gene was constructed, and then introduced into *E. coli* incapable of using sucrose as a carbon source, and the constructed recombinant microorganism was cultured in a medium containing sucrose as a single carbon source. As a result, it was found that the recombinant microorganism had the ability to metabolize sucrose. In still another aspect, the present invention relates to said recombinant vector, a recombinant microorganism transformed with the recombinant vector and capable of metabolizing sucrose, and a method for producing metabolites, biodegradable polymers or recombinant proteins using said recombinant microorganism.

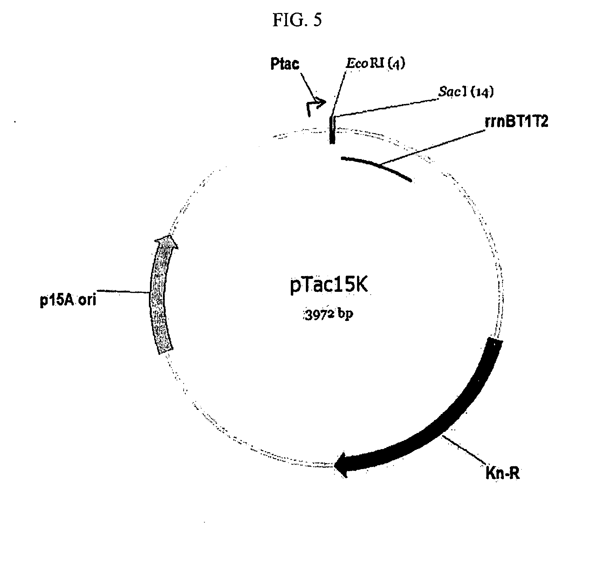

The recombinant vector may be a recombinant vector having a cleavage map of FIG. 5, but in addition to the pTac15K vector shown in FIG. 5, other known vectors may be used in the present invention. Furthermore, an expression cassette containing β-fructofuranosidase-encoding genes including the *sacC* gene may be inserted not only into expression vectors such as pTrc99A, pTac99A or pMAL series, but also into cloning vectors such as pACYC, pBluescript SK-, pBR322, pGEM series or pMB1, and the recombinant vectors may be applied in the present invention. In addition, it is also possible to use recombinant vectors known in the art to which the present invention pertains (Sambrook J & Russell D, Molecular Cloning: a laboratory manual, 3rd ed., Cold Spring Harbor Lab (CSHL) Press, New York, 2001). Moreover, vectors containing, in addition to a kanamycin-resistant gene, other several resistant genes known in the art, may also be used in the present invention.

In Examples, a microorganism incapable of using sucrose as a carbon source was transformed using a recombinant vector containing the *sacC, cscA* and *sacA* genes, thus constructing a recombinant microorganism capable of metabolizing sucrose. However, a recombinant microorganism capable of metabolizing sucrose may also be constructed by inserting the above genes into the chromosome of a microorganism incapable of using sucrose as a carbon source according to a method known in the art. In addition, in Examples of the present invention, only a specific *E*. *coli* strain was illustrated as a host microorganism incapable of using sucrose as a carbon source, it will be obvious to those skilled in the art that the same result as in the case of using the above *E*. *coli* strain can be obtained, even if the above genes are introduced not only into other *E*. *coli* strains, but also into host microorganisms incapable of using sucrose as a carbon source, including bacteria, yeasts and fungi.

As used herein, the term "metabolites" refers to a collection of intermediates and products which are produced through metabolic processes. The metabolites are classified into primary metabolites and secondary metabolites. For example, the present invention can be applied in various manners to a recombinant or genome-engineered microorganism incapable of using sucrose in order to produce biofuels, primary and secondary metabolites, biodegradable polymers and recombinant proteins. For the production of, for example, butanol (Atsumi et al., Nature., 451:7174, 2008), ethanol (Lindsay et al., Appl. Microbiol. Biotechnol, 43:70, 1995), and lactic acid (Zhou, Appl. Environ. Microbiol, 69:399 2003), and succinic acid and malic acid (Jantama et al., Biotechnol. Bioeng., 99:1140, 2008), amino acids (Park et al., PNAS, 104:7797, 2006; Lee et al., Molecular Systems Biology, 3:1, 2007), biodegradable polymers (Ahn et al., Appl. Environl. Microbiol., 66:3624, 2000; Park et al., Biomacromolecules, 2:248, 2001; Park et al., Biotechnol. Bioeng., 74:81, 2001), recombinant proteins (Jeong et al., Appl. Environl. Microbiol., 65:3027, 1999; Han et al., Appl. Environl. Microbiol., 69:5772, 2003), glucose has been used as a main carbon source in the prior art to produce the desired bioproducts; however, when the genes of the present invention are introduced into the known microbial strains, the desired bioporducts can be produced using sucrose as a carbon source.

In addition, a person skilled in the art can also easily apply the present invention to produce biodegradable polymers and recombinant proteins. In Examples of the present invention, the production of metabolites such as threonine was illustrated by way of example, but it will be obvious to a person skilled in the art that the present invention can also be easily applied for the production of biodegradable polymers, recombinant proteins and the like.

### Examples

Hereinafter, the present invention will be described in further detail with reference to examples. It is to be understood, however, that these examples are for illustrative purposes only and are not to be construed to limit the scope of the present invention.

Particularly, in Examples below, a specific *E. coli* strain was illustrated as a host strain incapable of metabolizing sucrose in order to express the genes of.the present invention, but it will be obvious to a person skilled in the art that, even if other *E*. *coli* strains or microorganisms of other species are used, metabolites including sucrose can be produced by introducing the genes of the present invention, which are involved in sucrose metabolism, into the microorganisms, and culturing the recombinant microorganisms.

### Example 1: Examination of the ability of ptsG, sacC and rbsK gene to metabolize sucrose

### 1.1: Isolation of ptsG, sacC and rbsK genes

In order to examine whether genes (*ptsG, sacC* and *rbsK*) according to the present invention are involved together in sucrose metabolism, the genes were isolated from *M succiniciproducens* MBEL55E (KCTC0769BP).

First, the DNA of *ptsG* (MS0784) was amplified by PCR using the genomic DNA of *M. succiniciproducens* MBEL55E (KCTC0769BP) as a template with primers of SEQ ID NOS: 11 and 12. Likewise, the DNAs of *sacC* (MS0909) and *rbsK* (MS1233) were amplified by PCR using a set of primers of SEQ ID NOS: 13 and 14 and a set of primers of SEQ ID NOS: 15 and 16, respectively, and overlapping PCR was performed using a mixture of the DNA fragments as a template with primers of SEQ ID NOS: 13 and 16. The *ptsG* (MS0784), *sacC* (MS0909) and *rbsK* (MS1233) are genes encoding sucrose phosphotransferase, sucrose-6-phosphate hydrolase, and fructokinase, respectively.
SEQ ID NO: 11: 5'-GGAATTCATGCTCGTTTTAGCTAGAATTGG
SEQ ID NO: 12: 5'-TCCGAGCTCTTACTATTCTTTTGCGTTAGCTCTTG
SEQ ID NO: 16: 5'-ACCTGCGGGTACCCTATTAGTTTGCTAAAAATTCCGCT

### 1.2: Construction of recombinant vector pMSscrIIA

In order to express the *Mannheimia*-derived genes, *ptsG* (MS0784), *sacC* (MS0909) and *rbsK* (MS1233), which encode sucrose phosphotransferase, sucrose-6-phosphate hydrolase and fructokinase, respectively, in *E*. *coli,* an expression vector was constructed in the following manner.

A DNA fragment containing the *sacC* (MS0909) and *rbsK* (MS1233) genes amplified in Example 1.1 was digested with the restriction enzymes *Sac*I and *Kpn*I and ligated into pTrc99A (Pharmacia Biotech., Uppsala, Sweden) digested with the same restriction enzymes, thus constructing pTrc99AsacCrbsK.

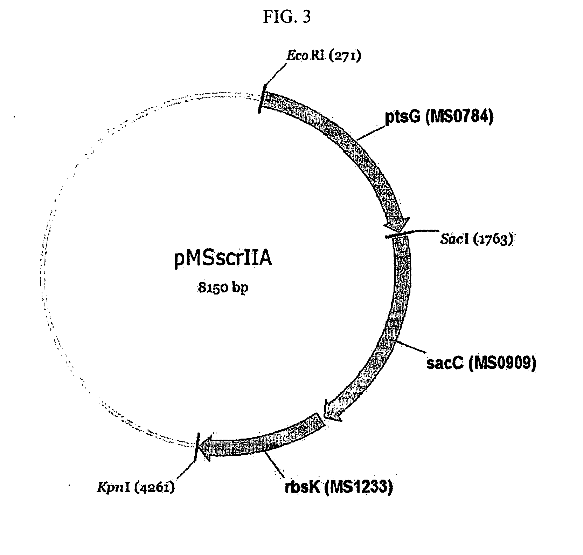

Then, the *ptsG* (MS0784) DNA fragment obtained in Example 1.1 was digested with *Eco*RI and *Sac*I and ligated into an expression vector pTrc99AsacCrbsK digested with the same restriction enzymes, thus constructing pTrc99AptsGsacCrbsK. The constructed vector was named "pMSscrIIA" (FIG. 3).

### 1.3: Construction of Escherichia coli W3110 pMSscrIIA and W3110 pTrc99A strains

The following experiment was carried out using, as a model microorganism, *E*. *coli* W3100 which is a substrain of *E*. *coli* K-12 known to be incapable of metabolizing sucrose.

*E. coli* W3110 was plated on LB solid medium and cultured at 37°C for 8 hours, and the colony was inoculated into 10 ml of LB liquid medium and cultured for 8 hours. The culture broth was inoculated into 100 mL of LB liquid medium at 1 %(v/v) and cultured in a shaking incubator at 37°C .

After about 2 hours, when the culture reached an OD₆₀₀ of about 0.30-0.35, it was left to stand on ice for 20 minutes to stop the growth of the cells. The culture broth was centrifuged at 4°C at 3,000 rpm for 15 minutes to collect the cells, and then the cells were suspended in 32 ml of RFI solution at 4°C. The suspension was centrifuged at 4°C at 3,000 rpm for 15 minutes to collect the cells. The cells were re-suspended in 8 ml of RFII solution, and then left to stand on ice for 15 minutes. Finally, the re-suspension was dispensed in an amount of 100 uℓ/well and stored at -70°C . The composition of RFI solution consisted of 100 mM RbCl, 50mM MnCl₂-4H₂O, 0.1 M CH₃COOK, 10 mM CaCl₂ and 15 %(w/v) glycerol and was adjusted to pH of 5.8 by the addition of 0.2 M acetate. The RFII solution consisted of 10 mM MOPS, 10 mM RbCl, 100 mM CaCl₂ and 15% (w/v) glycerol and was adjusted to pH of 6.8 by the addition of NaOH.

The expression vector pMSscrIIA constructed in Example 1.2 or pTrc99A (Pharmacia Biotech., Uppsala, Sweden) as a control was added to the *E*. *coli* W3110 strain, and then the strain was subjected to heat-shock transformation at 42 °C for 90 seconds, thus transforming the strain. After the heat-shock transformation, 0.8 ml of LB liquid medium was added to the strain and the strain was cultured in a shaking incubator at 37°C for 1 hour.

The culture broth was plated on LB solid medium containing antibiotic ampicillin (final concentration: 50 ug/L) and cultured at 37°C for 12 hours or more. The formed *E*. *coli* W3110 pMSscrIIA and *E*. *coli* W3110 pTrc99A colonies were inoculated into LB liquid medium and cultured at 37°C for 8 hours or more. In order to confirm whether the vector was successfully introduced, the vector was isolated from the cultured strain using GeneAll^{R} Plasmid SV (GeneAll Biotechnology, Korea) and subjected to electrophoresis. *E*. *coli* W3110 pMSscrIIA strain was sequenced in Solgent Co. (Korea) using primers of SEQ ID NOS: 17 to 24, thus examining whether the base sequences of the strain were consistent with the base sequences of the genes.
SEQ ID NO: 17: 5'-GGAAACAGACCATGGAATTC
SEQ ID NO: 18: 5'-CCGCAAAAGATTTATTCGAAGAAG
SEQ ID NO: 19: 5'-CCTGGTTATATGATACTTTAGG
SEQ ID NO: 20: 5'-TAGTGCTGGGCGCAAGAGCTAACG
SEQ ID NO: 21: 5'-ACCAGTGGGCGATAAAATCG
SEQ ID NO: 22: 5'-TGATCAAGGTTTCGATTTCT
SEQ ID NO: 23: 5'-TTTTCCTGAATGACGGCGAA
SEQ ID NO: 24: 5'-CGATCTGCCGCAATTTCAAG

### 1.4: Examination of the ability of recombinant E. coli to metabolize sucrose

*E. coli* W3110 pMSscrIIA and *E*. *coli* W3110 pTrc99A colonies on solid medium, constructed in Example 1.3, were inoculated into a M9 minimal medium containing 5 g/L of sucrose as a single carbon source and were cultured at 37°C for 16 hours. Then, the culture broth was inoculated into 100 ml of a M9 minimal medium containing sucrose at 3 %(v/v), and then cultured at 37°C. Herein, as an antibiotic, ampicillin was added to a final concentration of 50 µg/L. The M9 minimal medium consisted of 33.9 g/L of Na₂HPO₄, 15 g/L of KH₂PO₄, 2.5 g/L of NaCl, 5 g/L of NH₄Cl and 0.36 g/L of MgSO₄. The concentration of cells in the culture medium was measured as OD₆₀₀ using a spectrophotometer. During the culture period, a sample was periodically collected, the collected sample was centrifuged at 13,000 rpm for 5 minutes, and then the concentration of sucrose in the supernatant was analyzed by high-performance liquid chromatography (HPLC).

As a result, as shown in Table 1, *E. coli* W3110 pTrc99A strain could not grow in the M9 minimal medium containing sucrose as a single carbon source, but *E. coli* W3110 pMSscrIIA strain showed an excellent ability to metabolize sucrose. *E. coli* W3110 pMSscrIIA strain metabolized about 2.2 g/L of sucrose for 19 hours, indicating an increase in biomass of 3.12 based on OD₆₀₀, and produced 0.67 g/L of acetic acid as a byproduct. Thus, it was confirmed that, when a microorganism contains all the *ptsG, sacC* and *rbsK* genes, it shows an excellent ability to metabolize sucrose.

**Table 1**

| Strain | Plasmid | Growth in M9 minimal medium + 5 g/L sucrose | Sucrose utilizing phenotype | Sucrose concentration (g/L) | |
|---|---|---|---|---|---|
| | | | | 0 h | 19 h |
| *E. coli* W3110 | pTrc99A | - | scr- | 5.23 | 5.22 |
| *E. coli* W3110 | pMSscrIIA | + | scr+ | 6.73 | 4.53 |

As described above, when either the sucrose phosphotransferase gene or a combination of the phosphotransferase sucrose gene with the sucrose-6-phosphate hydrolase gene was introduced into the microorganism incapable of metabolizing sucrose, the microorganism had the ability to metabolize sucrose and, in addition, produced acetic acid as a metabolite using sucrose as a carbon source.

Accordingly, any person skilled in the art can also easily apply the present invention for the production of, in addition to acetic acid, lactic acid, succinic acid, ethanol, biofuel and bioenergy containing biobutanol, biodegradable polymers and recombinant proteins.

### Example 2: Examination of the ability of each of pstG gene and sacC gene to metabolize sucrose

### 2.1: Construction of recombinant vector for examining the ability of pstG gene and sacC gene to metabolize sucrose

In order to examine whether the *ptsG* and *sacC* genes of the present invention are involved alone in the ability to metabolize sucrose, a vector (pSacHR06ptsG) for deletion of *ptsG* (MS0784) and a vector (pSacHR06sacC) for deletion of *sacC* (MS0909) were constructed and subjected to a knock-out experiment.

First, in order to disrupt the sucrose phosphotransferase gene *(ptsG)* by homologous recombination, a gene exchange vector was constructed in the following manner. The left homologous arm region was amplified using the genomic DNA of *Mannheimia succiniciproducens* MBEL55E (KCTC0769BP) as a template with primers of SEQ ID NOS: 25 and 26; and the right homologous arm region was amplified using primers of SEQ ID NOS: 27 and 28; a DNA fragment containing an antibiotic marker and a mutant lox site was amplified using a pECmulox vector (Kim et al., FEMS Microbiol. Lett., 278:78, 2008) as a template with primers of SEQ ID NOS: 29 and 30. These three DNA fragments were amplified by overlapping PCR using primers of SEQ ID NOS: 25 and 28.
SEQ ID NO: 25: 5'-ATATCTGCAGCCGGCATTAAATATTAGTCAAC
SEQ ID NO: 26: 5'-CGTTCTAACGGAGGTTGAAAACTGCCCTTT
SEQ ID NO: 27: 5'-GTCTCCCTATCACGCCGTTATTTTCATTATT
SEQ ID NO: 28: 5'-ATTAGTCGACACCATCCCCACGGAATACAT
SEQ ID NO: 29: 5'-TTTCAACCTCCGTTAGAACGCGGCTACAAT
SEQ ID NO: 30: 5'-TAACGGCGTGATAGGGAGACCGGCAGATCC

The final DNA fragment thus amplified was digested with the restriction enzymes *Pst*I and *Sal*I and cloned into pSacHR06 vector (Park et al., PNAS, 104:7797, 2007) digested with the same enzymes, thus constructing pSacHR06ptsG. In addition, pSacHR06sacC was constructed in the same manner as described above using primers of SEQ ID NOS: 31 to 36.
SEQ ID NO: 31: 5'-ATACACTGCAGTTATGCAATTTATCGCACCC
SEQ ID NO: 32: 5'-AATCTGCTCTGATGCGGTCGTGAAATGCTTCCA
SEQ ID NO: 33: 5'-CACAGAATCAGGACAAATGGCATTCAATGCTG
SEQ ID NO: 34: 5'-ATACTGTCGACTCAATGGCATATGCAGCG
SEQ ID NO: 35: 5'-AAGCATTrCACGACCGCATCAGAGCAGATTGTACTGAGAG
SEQ ID NO: 36: 5'-TTGAATGCCATTTGTCCTGATTCTGTGGATAACCGTATTAC

### 2.2: Construction of M. succiniciproducens MptsG and M. succiniciproducens MsacC strains

Using each of the exchange vector pSacHR06ptsG for deletion of the *ptsG* gene and the exchange vector pSacHR06sacC for deletion of the *sacC* gene, constructed in Example 2.1, the genes were deleted from the genome of *M. succiniciproducens* MBEL55E (KCTC0769BP) according to the method reported by Kim et al. (FEMS Microbiol. Lett., 278:78, 2008), thus constructing mutant strains, *M succiniciproducens* MptsG and *M succiniciproducens* MsacC.

Specifically, *M. succiniciproducens* MBEL55E (KCTC0769BP) was plated on a LB-glucose solid medium containing 10 g/L of glucose and was cultured at 37 °C for 36 hours. Then, the colony was inoculated into 10 ml of LB-glucose liquid medium and cultured for 12 hours. The sufficiently grown cell culture was inoculated into 100 ml of LB-glucose liquid medium at 1% (v/v) and cultured in a shaking incubator at 200 rpm at 37°C.

When the culture broth reached OD₆₀₀ of about 0.3-0.4 after about 4-5 hours, it was centrifuged at 4°C at 4,500 rpm for 20 minutes to collect the cells, and then the cells were re-suspended in 200 ml of 10% glycerol solution at 4°C. The re-suspension was centrifuged at 4 °C at 5,500 rpm for 20 minutes to collect the cells. The re-suspension process was repeated twice while reducing the glycerol solution to half, and then the cells were re-suspended in glycerol solution at a volume ratio 1:1 to obtain a cell concentrate. The cell concentrate was mixed with each of the gene exchange vectors pSacHR06ptsG or pSacHR06sacC constructed in Example 2.1, and then was subjected to electroporation in the conditions of 2.5 kV, 25 µF and 400 ohms, thus introducing each of the genes into the cultured *M. succiniciproducens.* After the electroporation, the 1 ml of LB-glucose liquid medium was added to the strain and then the strain was cultured in a shaking incubator at 200 rpm at 37 °C for 1 hour. The culture broth was plated on a LB-glucose solid medium containing antibiotic chloramphenicol (final concentration: 6.8 µg/L) and was cultured at 37°C for 48 hours or more. In order to select a colony where only double crossover occurred, the formed colonies were streaked on LB-sucrose medium (LB medium containing 100 g/L sucrose) containing chloramphenicol (final concentration: 6.8 µg/L). After 24 hours, the formed colonies were streaked again on the same plate.

The colony (mutant) formed on the plate was cultured in LB-glucose liquid medium containing an antibiotic, and a genomic DNA was isolated from the cultured strain by the method described in Rochelle et al. (FEMS Microbiol. Lett., 100:59, 1992). PCR was performed using the isolated mutant genomic DNA as a template, and the PCR product was electrophoresed to confirm the deletion of *ptsG* and *sacC* in each of the mutant strains.

In order to confirm the deletion of *ptsG* in the MptsG strain, PCRs were performed using a set of primers of SEQ ID NOS: 37 and 38 and a set of primers of SEQ ID NOS: 39 and 40, respectively. In order to confirm the deletion of *sacC* in the MsacC strain, PCRs were performed using a set of primers of SEQ ID NOS: 39 and 40 and a set of primers of SEQ ID NOS: 41 and 42, respectively.
SEQ ID NO: 37: 5'-CGGGGCGAAAGTGATTGAGA
SEQ ID NO: 38: 5'-AATTGCCGCCTGGGTATTGG
SEQ ID NO: 39: 5'-ACCTTTACTACCGCACTGCTGG
SEQ ID NO: 40: 5'-GCGGGAGTCAGTGAACAGGTAC
SEQ ID NO: 41: 5'-GATCTTGAGTCCGTAAAACAGGCTT
SEQ ID NO: 42: 5'-TTCCGCTCAAGCCATTGTAGTG

### 2.3: Comparison of growth between MptsG strain, MsacC strain and parent strain (MBEL55E)

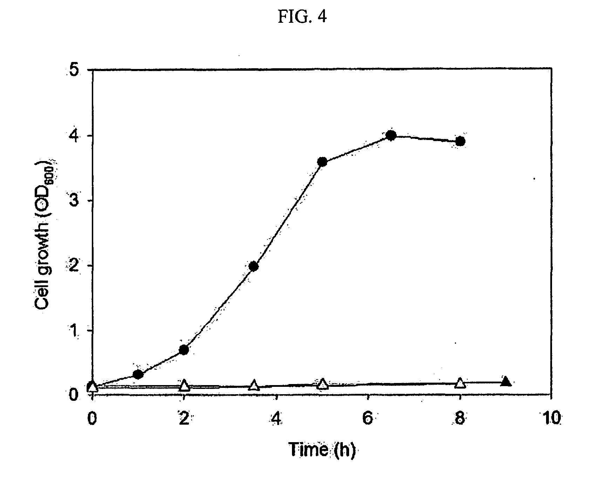

Each of the recombinant MptsG and MsacC strains constructed in Example 2.2 was cultured in BHI (Bacto^{™} Brain Heart Infusion; Becton, Dickinson and Company, Sparks, MD) for about 8 hours, and 10 ml of the culture broth was inoculated into 300 ml of MH5S culture medium (per liter: 2.5 g of yeast extract, 2.5 g of polypeptone, 1 g of NaCl, 8.7 g of K₂HPO₄, 10 g of NaHCO₃, 0.02 g of CaCl₂2H₂O, 0.2 g of MgCl₂6H₂O and 10 g of sucrose), and the growth curves of the strains were compared with the growth curve of the parent strain MBEL55E cultured in the same conditions. FIG. 4 is a set of growth curves of the MBEL55E (●), MptsG (▲) and MsacC (Δ) strains, measured as OD₆₀₀. As shown in FIG. 4, the growth ability of the parent strain (MBEL55E) in the sucrose medium substantially disappeared when each of the genes was deleted from the strain. This indicates that each of the genes is essential for growth of the strain in the sucrose medium.

Meanwhile, the *rbsK* gene encoding fructokinase (RbsK, MS1233) was subjected to the same experiment as described above, a change in growth such as a decrease in growth rate was not observed. Also, in measurement results for enzymatic activity, the parent strain and the *rbsK*-deleted strain showed no great difference in enzymatic activity therebetween and had enzymatic activity levels which were very lower than that of general fructokinase. Namely, the two strains showed negligible enzymatic activities. Based on such results, it is inferred that the *rbsK* gene does not encode fructokinase or it has a very weak activity, even though it encodes fructokinase.

### 2.4: Comparison of enzymatic activities of MptsG and MsacC strains and parent strain

To measure the enzymatic activity of sucrose PTS, the methods of Jacobson et al. (J. Biol. Chem., 254:249, 1979) and Lodge et al. (Infect. Immun., 56:2594, 1988) were used.

First, to permeabilize cells, 1 ml of the cell culture broth at an OD₆₀₀ value of about 1.2 was washed with TDM buffer (50 mM Tris/HCl, 10 mM MgCl₂, 1 mM DTT; pH 7.5) and re-suspended in 1 ml of the same buffer, 0.01 ml toluene was added thereto, and the cell solution was strongly agitated for 45 seconds. The agitated cell solution was centrifuged at 12,000×g, and the collected cells were washed twice with TDM buffer. This procedure was repeated once more, and the resulting cells were re-suspended in 50 µℓ of TDM buffer, thus preparing permeabilized cells. PEP-dependent sucrose phosphorylation was performed by adding 5µℓ of the permeabilized cell suspension to 100µℓ of a reaction mixture containing 25 mM Tris/HCl (pH 8.0), 1 mM DTT, 5 mM MgCl₂, 10 mM KF and 1µCi[U-¹⁴C] sucrose, and measuring the difference in reaction between the mixture containing 1 mM PEP and the mixture not containing PEP. The mixture was allowed to react at 37 °C for 10 minutes, and 1 mL of cold water was added thereto to stop the reaction. The final reaction product was passed through a DEAE filter disk (Whatman, DE81) on a filter system and washed with a 10-fold volume of cold water, and then the radioactivity thereof was measured according to a known method using the Beckman LS6500 liquid scintilation counter (Beckman, Ramsey, MN). The activity of sucrose-6-phosphate hydrolase was measured by some modification of the method of Martin et al. (Appl. Environ. Microbiol., 53:2388, 1987). 20 ml of the cell culture at OD₆₀₀ value of about 1 was permeabilized in the same manner as in the measurement of sucrose PTS activity, and finally re-suspended in 1 mL of TDM buffer, thus preparing permebilized cells. PEP-dependent sucrose phosphorylation was performed by adding 30µℓ of the permeabilized cells to 300µℓ of a reaction mixture containing 50 mM sodium-potassium phosphate buffer (pH 7.2), 5 mM MgCl₂, 4 mM sucrose, 0.8 mM NADP and 6.4 U glucose-6-phosphate dehydrolase, and measuring the difference in reaction between the mixture containing 10 mM PEP and the mixture not containing PEP.

The activities of the mutant strains from which each of the *ptsG* and *sacC* genes has been removed and the activity of the parent strain were measured, and the measurement results are shown in Table 2 below. The results reveal that the sucrose PTS enzyme encoded by MS0784(*ptsG*) has PTS activity and that the sucrose-6-phosphate hydrolase enzyme encoded by MS0909(*sacC*) has glycolytic functions in cells.

**Table 2**

| Enzymes | Strains | Culture medium^{a} | Specific enzyme activity (mU/mg)^{b} | Relative activity (%)^{c} |
|---|---|---|---|---|
| Sucrose phosphotransferase | MBEL55E | MH5S* | 3.7 | 100.0 |
| | | BHI | 1.4 | 37.8 |
| | MptsG | BHI | 0.098 | 2.6 |
| Sucrose-6-phosphate hydrolase | MBEL55E | MH5S* | 18.3 | 100.0 |
| | | BHI | 20.4 | 111.5 |
| | MsacC | BHI | 1.7 | 9.3 |

| | | | | |
|---|---|---|---|---|
| ^{a}BHI, BactoTMBrainHeartInfusion (Becton, DickinsonandCompany, Spark, MD); the MH5S medium composition contains: per liter, 2.5 g of yeast extract, 2.5 g of polypeptone, 1 g of NaCl, 8.7g of K₂HPO₄, 10 g of NaHCO₃, 0.02 g of CaCl₂·H₂O, 0.2 g of MgCl₂·6H₂O and 10 g of sucrose. ^{b}Activity of sucrose phosphotransferase was expressed in a unit of mU/mg converted from cpm (count per minute) measured through a liquid scintillation counter. ^{c}Relative activity was determined by calculating the remaining values for 100% for the culture broth indicated by the symbol *. | | | | |

### Example 3: Isolation of novel genes encoding enzymes involved in sucrose metabolism

Genes encoding β-fructofuranosidase, including *sacC,* confirmed to have the ability to metabolize sucrose on the basis of the results of Example 2, were isolated from each of *Mannheimia, E. coli* and *Bacillus subtilis* in the following manner.

### 3.1: Isolation of gene encoding sucrose-6-phosphate hydrolase derived form Mannheimia

First, the DNA of the *sacC* (MS0909) gene was amplified by PCR using the genomic DNA of *M. succiniciproducens* MBEL55E (KCTC0769BP) as a template with primers of SEQ ID NOS: 43 and 44. The *sacC* (MS0909) gene is a gene (SEQ ID NO: 4) encoding α-fructofuranosidase (sucrose-6-phosphate hydrolase).
SEQ ID NO 43: 5'-ACTGAGCCATGGCGAAAATCAATAAAGTAGATC-3'
SEQ ID NO 44: 5'-TGATCCGAGCTCCTATTATTCCAGTGTTCCCGCC-3'

### 3.2: Isolation of gene encoding invertase derived from E. coli

The DNA of the *cscA* gene was amplified by PCR using the genomic DNA of *E*. *coli* W as a template with primers of SEQ ID NOS: 45 and 46. The *cscA* is a gene (SEQ ID NO: 8) encoding β-fructofuranosidase (invertase).
SEQ ID NO 45: ACTCCGGAATTCATGACGCAATCTCGATTGCA
SEQ ID NO 46: ACCTGCGAGCTCCCGTTGTTCCACCTGATATTATG

### 3.3: Isolation of gene encoding sucrose-6-phosphate hydrolase derived from Bacillus subtilis

The DNA of the *sacA* gene was amplified by PCR using the genomic DNA of *Bacillus subtilis* as a template with primers of SEQ ID NOS: 47 and 48. The *sacA* is a gene (SEQ ID NO: 10) encoding β-fructofuranosidase (sucrose-6-phosphate hydrolase).
SEQ ID NO 47: GCATAGAATTCATGACAGCACATGACCAGGAGCT
SEQ ID NO 48: GCATAGAGCTCCTACATAAGTGTCCAAATTCCGACATTC

### Example 4: Construction of recombinant vectors

### 4.1: Preparation of pTac15K

A pTac15K vector was constructed by inserting the trc promoter and transcription terminator regions of pKK223-3 (Pharmacia Biotech., Uppsala, Sweden) into pACYC177 (NEB, Beverly, MA, USA). pTac15K is a expression vector for constitutive expression and has a structure shown in a cleavage map of FIG. 5.

### 4.2: Construction of ptac15KsacC

In order to express the gene *sacC* (MS0909) encoding *Mannheimia*-derived β-fructofuranosidase (sucrose-6-phosphate hydrolase) in *E. coli,* an expression vector was constructed in the following manner.

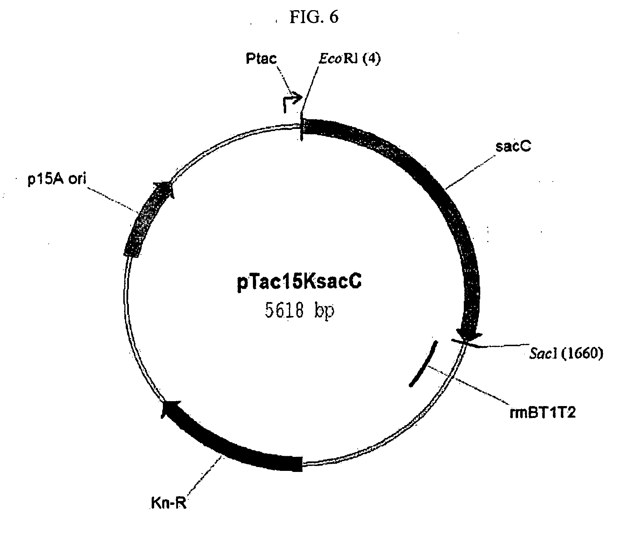

According to a known molecular engineering method, the *sacC* (MS0909) gene-containing PCR fragment amplified in Example 3.1 was digested with *Eco*RI and *Sac*I and ligated into pTac15K digested with the same enzymes, thus constructing pTac15KsacC (FIG. 6). The constructed vector was sequenced in Solgent Co. (Korea) using primers of SEQ ID NOS: 49 to 52, thus examining whether the base sequence of the gene introduced into the vector was consistent with that of the *sacC* (MS0909) gene.
SEQ ID NO 49: 5'-CCCGTTCTGGATAATGTTTT-3'
SEQ ID NO 50: 5'-AAAGTCACGGTTGTTATTCC-3'
SEQ ID NO 51: 5'-CATTTAATGCCGCTCATATT-3'
SEQ ID NO 52: 5'-ACCGCTCAATTATTGAGATT-3'

### 4.3: Construction of recombinant vector pTac15KEWcscA

According to a known molecular engineering method, the DNA fragment obtained in Example 3.2 were digested with *EcoR*I and *Sac*I and ligated into pTac15K digested with the same enzymes, thus constructing pTac15KEWcscA.

### 4.4 Construction of recombinant vector pTac15KBSsacA

According to a known molecular engineering method, the DNA fragment obtained in Example 3.3 was digested with *EcoR*I and *Sac*I and ligated into pTac15K digested with the same enzymes, thus constructing pTac15KBSsacA.

### Example 5: Construction of recombinant strains

### 5.1: Construction of Escherichia coli W3110 pTac15KsacC strain

The following experiment was carried out using, as a model microorganism, *E*. *coli* W3110 which is a substrain of *E*. *coli* K-12 known to be incapable of metabolizing sucrose. *E. coli* W3110 strain was plated on LB solid medium and cultured at 37 °C for 8 hours, and then the colony was inoculated into 10 ml of LB liquid medium and cultured for 8 hours. The culture broth was inoculated into 100 ml of LB liquid medium at 1 %(v/v) and cultured in a shaking incubator at 37°C.

When the culture broth reached OD₆₀₀ of about 0.30-0.35 after about 2 hours, it was left to stand on ice for 20 minutes to stop the growth of the cells. The culture broth was centrifuged at 4 °C at 3,000 rpm for 15 minutes to collect the cells, and then the cells were suspended in 32 ml of RFI solution at 4°C. The cell suspension was centrifuged at 4 °C at 3,000 rpm for 15 minutes to collect the cells. The collected cells were re-suspended in 8 ml of RFII solution, and then let to stand on ice for 15 minutes. Finally, the re-suspension was dispensed in an amount of 100 µℓ/well and stored at -70°C. The composition of the RFI solution consisted of 100 mM RbCl, 50 mM MnCl₂-4H₂O, 0.1 M CH₃COOK, 10 mM CaCl₂ and 15 %(w/v) glycerol and was adjusted to a pH of 5.8 by the addition of 0.2 M acetate. The RFII solution consisted of 10 mM MOPS, 10 mM RbCl, 100 mM CaCl₂ and 15% (w/v) glycerol and was adjusted to a pH of 6.8 by the addition of NaOH.

The expression vector constructed in Example 2.2 or pTac15K (Pharmacia Biotech., Uppsala, Sweden) as a control was added to *E*. *coli* W3110 strain, and then the strain was subjected to heat-shock transformation at 42 °C for 90 seconds, thus transforming the strain. After the heat-shock transformation, 0.8 ml of LB liquid medium was added to the strain and then strain was cultured in a shaking incubator at 37°C for 1 hour.

The culture broth was plated on a LB solid medium containing antibiotic kanamycin (final concentration: 50 µg/L) and cultured at 37 °C for 12 hours or more. The formed *E*. *coli* W3110 pTac15K and *E*. *coli* W3110 pTac15KsacC colonies were inoculated into LB liquid medium and cultured at 37 °C for 8 hours or more. In order to confirm whether the vector was successfully introduced into the strain, the vector was isolated from the cultured strain using GeneAll^{R} Plasmid SV (GeneAll Biotechnology, Korea) and subjected to electrophoresis.

### 5.2: Construction of E. coli W3110 pTac15KEWcscA and E. coli W3110 pTac15KBSsacA strains

According to the same method as described in Example 5.1, *E*. *coli* W3110 which is a substrain of *E*. *coli* K-12 known to be incapable of metabolizing sucrose was transformed using each of the pTac15KEWcscA and pTac15KBSsacA vectors constructed in Examples 4.3 and 4.4, and whether the vector was successfully introduced was confirmed by electrophoresis.

### Example 6: Examination of sucrose-metabolizing ability and growth of recombinant E. coli

Each of *E*. *coli* W3110 pTac15KsacC and *E*. *coli* W3110 pTac15K colonies on solid media, constructed in Example 5.1, was inoculated into 10 ml of LB medium and cultured at 37 °C for 8 hours. Then, the cells were inoculated into 100 ml of a M9 minimal medium (containing 10 g/L of sucrose) at 5 %(v/v) and cultured at 37 °C. Herein, as an antibiotic, kanamycin was added to a final concentration of 50 µg/L. The LB medium consisted of 10 g/L of tryptone, 10 g/L of NaCl and 5 g/L of yeast extract, and the M9 minimal medium consisted of 33.9 g/L of Na₂HPO₄, 15 g/L of KH₂PO₄, 2.5 g/L of NaCl, 5 g/L of NH₄Cl and 0.36 g/L of MgSO₄. The concentration of cells in the culture broth was measured as OD₆₀₀ using a spectrophotometer. During the culture period, a sample was periodically collected, and the collected sample was centrifuged at 13,000 rpm for 5 minutes. Then, the concentrations of sucrose and metabolites in the supernatant were analyzed by high-performance liquid chromatography (HPLC).

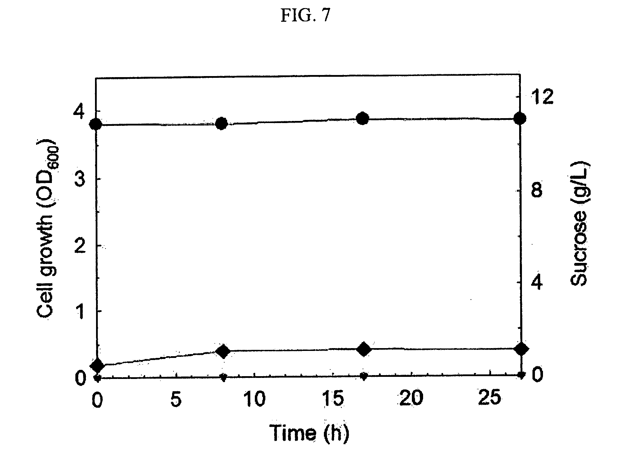

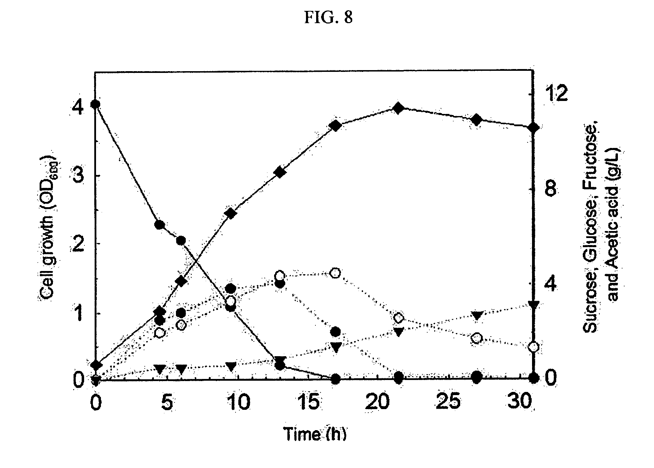

As a result, as shown in FIGS. 7 and 8 and Table 3, *E*. *coli* W3110 pTac15K strain could not grow in the M9 minimal medium containing sucrose as a single carbon source, but *E*. *coli* W3110 pTac15KsacC strain showed an excellent ability to metabolize sucrose. *E*. *coli* W3110 pTac15KsacC strain metabolized 11.08 g/L of sucrose for 17 hours and showed an increase in biomass of 3.71 based on OD₆₀₀. This increase in biomass corresponds to a biomass amount of about 1.37 g/L in view of the conversion factor (1 OD₆₀₀ = 0.37 g/L DCW) of OD₆₀₀ and dry cell weight (DCW, g/L) of conventional *E*. *coli.* In addition, it could be observed that 1.42 g/L of acetic acid was produced, 2.01 g/L of glucose and 4.51 g/L of fructose remained, and the two saccharides were gradually consumed with the passage of time.

**Table 3**

| Strains | Plasmids | Growth in M9 minimal medium +10g/L sucrose | Sucrose utilizing phenotype | Sucrose conc. (g/L) | |
|---|---|---|---|---|---|
| | | | | 0 h | 17 h |
| *E. coli* W3110 | pTac15K | - | Scr- | 11.00 | 11.12 |
| *E. coli* W3110 | pTac15KsacC | + | Scr+ | 11.08 | 0.00 |

In the growth curve of the W3110 pTac15K strain (FIG. 7), a slight increase in OD at 8 hours after inoculation is believed to be attributable to the components of the medium into which it was inoculated at 5 %(v/v). The results of LC analysis of the W3110 pTac15K strain indicated that the stain neither degrade sucrose nor grow using sucrose as a single carbon source.

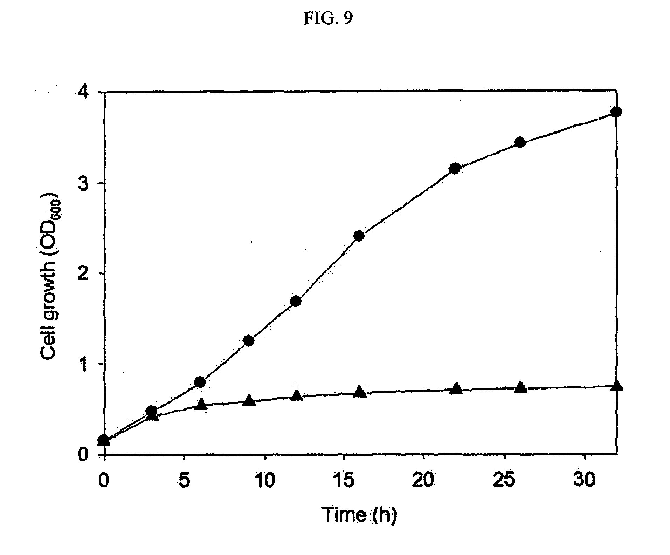

Moreover, the recombinant strains *E*. *coli* W3110 pTac15KEWcscA and *E*. *coli* W3110 pTac15KBSsacA transformed in Example 5.2 were cultured using sucrose as a single carbon source in the same manner as in the case of *E*. *coli* W3110 pTac15KsacC strain. As a result, as shown in FIG. 9, the strains showed an excellent ability to grow. Also, in the case of *E*. *coli* W3110 pTac15KEWcscA, sucrose decreased from 7.77 g/L at the initial stage to 1.82 g/L after 48 hours, and in the case of *E*. *coli* W3110 pTac15KBSsacA strain, sucrose decreased from 8.49 g/L at the initial stage to 7.98 g/L after 48 hours. This suggests that these strains can grow by metabolizing sucrose.

### Example 7: Production of metabolites in recombinant E. coli using sucrose as carbon source

As described above, when the sucrose-6-phosphate hydrolase derived from *M succiniciproducens* was introduced into the microorganism incapable of growing using sucrose as a carbon source, the microorganism could grow using sucrose as a single carbon source. Namely, when a microorganism incapable of using sucrose as a carbon source is treated such that it can cultured in a minimal medium using sucrose as a single carbon source, existing systems for producing various bioporducts (e.g., primary and secondary metabolites, recombinant proteins and biodegradable polymers) can be applied to the present invention.

Accordingly, this Example shows the production of various metabolites using sucrose in anaerobic conditions.

*E. coli* W3110 pTac15KsacC strain constructed in Example 5.1 was inoculated into 10 ml of LB medium and cultured at 37 °C for 8 hours, and then the culture broth was inoculated into 200 mL of LB medium and cultured at 37 °C for 8 hours. Then, the culture broth was inoculated into a 2.5-L volume fermentor (New Brunswick System, BioFlo 3000). A culture medium in the fermentor consisted of an R/2 minimal medium containing 20 g/L of sucrose, and 100% CO₂ was introduced into the fermentor at a rate of 0.5 vvm under operating conditions of pH 6.8, 37 °C and 200 rpm. As an antibiotic, kanamycin was added to a final concentration of 50 µg/L. The R/2 medium consisted of 6.75 g/L of KH₂PO₄, 2 g/L of (NH₄)₂HPO₄, 0.85 g/L of C₆H₈O₇H₂O, 0.7 g/L of MgSO₄7H₂O and 5 ml/L of trace metal solution (10 g/L FeSO₄7H₂O 2 g/L CaCl₂, 2.2 g/L ZnSO₄7H₂O 0.54 g/L MnSO₄5H₂O, 1 g/L CuSO₄5H₂O, 0.1 g/L NH₄Mo₇O₂₄7 H₂O, 0.02 g/L Na₂B₄O₇10H₂O, and 5 mL HCl). The concentration of cells in the culture broth was measured as OD₆₀₀ using a spectrophotometer, and during the culture period, a sample was periodically collected, the collected sample was centrifuged at 13,000 rpm for 5 minutes, and then the concentrations of sucrose and metabolites in the supernatant were analyzed by high-performance liquid chromatography (HPLC).

As a result, as shown in FIG. 10 and Table 4, acetic acid, formic acid, lactic acid, succinic acid and ethanol could be successfully produced. In the above-described anaerobic conditions for 52.5 hours, 22.13 g/L of sucrose and saccharides derived therefrom were completely consumed and, as a result, 4.49 g/L of acetic acid, 3.74 g/L of formic acid, 4.19 g/L of lactic acid, 5.10 g/L of succinic acid and 2.66 g/L of ethanol were produced at yields of 0.20, 0.17, 0.19, 0.23, and 0.12 g/g of sucrose, and the total sum of these yields was 0.91 g/g of sucrose which significantly approached the theoretical value. Also, OD₆₀₀ at 52.5 hours after the start of the culture was 2.7 which corresponded to a g DCW of 0.999 in view of the above-described conversion factor. The calculated specific yield indicating yield per strain weight is shown in Table 4 below. The results of Table 4 indicate that, when the sucrose-6-phosphate hydrolase is introduced alone, various metabolites can be successfully produced at high yield and high specific yield using sucrose.

**Table 4**

| Sucrose and products | Initial conc. | Final conc. | Yield (g/g sucrose) | Specific yield (g/g DCW) |
|---|---|---|---|---|
| Sucrose | 22.13 | 0 | - | - |
| Acetic acid | 0 | 4.49 | 0.20 | 4.49 |
| Formic acid | 0 | 3.74 | 0.17 | 3.74 |
| Lactic acid | 0 | 4.19 | 0.19 | 4.19 |
| Succinic acid | 0 | 5.10 | 0.23 | 5.11 |
| Ethanol | 0 | 2.66 | 0.12 | 2.66 |
| Total | 22.13 | 20.18 | 0.91 | 20.19 |

### Example 8: Application to metabolically engineered useful bioproducts through application to threonine-producing strain

The following experiment was carried out using a threonine-producing strain in order to illustrate that when the genes of the present invention are introduced into a genome-engineered or recombinant *E*. *coli* strain, the strain can also produce bioproducts using sucrose instead of other existing carbon sources. Specifically, the following experiment was carried out using, as a model, a genome-engineered and recombinant TH28C pBRThrABCR3 (Lee et al., Molecular Systems Biology, 3:149, 2007) metabolically modified based on *E*. *coli* W3110.

According to the same method as described in Example 5, the TH28C pBRThrABCR3 strain was transformed with the plasmid pTac15KsacC constructed in Example 4, thus constructing TH28C pBRThrABCR3 pTac15KsacC.

The constructed TH28C pBRThrABCR3 pTac15KsacC strain was inoculated into 10 mL of LB medium and cultured at 31 °C for 12 hours, and the culture broth was inoculated into 50 mL of LB medium and cultured at 31 °C for 12 hours. Then, the culture broth was inoculated into a 2.5-L volume fermentor (New Brunswick System, BioFlo 3000). The culture medium contained in the fermentor consisted of a TPM2 medium containing 20 g/L of sucrose and was maintained at an air saturation level of more than 40% by supplying air having a dissolved oxygen concentration of 1 vvm under conditions of pH of 6.5 and temperature of 31 °C and automatically increasing revolution speed (rpm) to 1000. As antibiotics, chloramphenicol, kanamycin and ampicillin were added to final concentrations of 30 µg/L, 40 µg/L and 50 µg/L, respectively. The TPM2 medium consisted of 2 g/L of yeast extract, 2 g/L of MgSO₄˙7H₂O, 2 g/L of KH₂PO₄, 10 g/L of (NH₄)₂SO₄, 0.2 g/L of L-methionine, 0.2 g/L of L-lysine, 0.05 g/L of L-isoleucine, and 10 ml/L of trace metal solution (10 g/L FeSO₄7H₂O, 2 g/L CaCl₂, 2.2 g/L ZnSO₄7H₂O, 0.54 g/L MnSO₄5H₂O, 1 g/L CuSO₄5H₂O, 0.1 g/L NH₄Mo₇O₂₄7H₂0, 0.02 g/L Na₂B₄O₇10H₂O, and 5 mL HCl). The concentration of cells in the culture broth was measured as OD₆₀₀ using a spectrophotometer, and the periodically collected sample was centrifuged at 13,000 rpm for 5 minutes, and then the concentrations of sucrose and metabolites in the supernatant was analyzed by high-performance liquid chromatography (HPLC). For analysis of amino acids, 5 ml of the culture broth was subjected to centrifugation and filtration processes, and then separated in Science Lab Center Co. (Daejeon, Korea) using a cation separation column (LCA K06/Na 1.6150mm; SykamGmbH, Eresing, Germany). Then, the separated sample was analyzed using the Sykam S433 amino-acid analyzer.

As a result, as shown in Table 5 below, 4.7 g/L of threonine could be successfully produced using sucrose.

**Table 5**

| | Initial value | Final value (after 15h culture) |
|---|---|---|
| OD₆₀₀ | 0.4 | 18.0 |
| Sucrose | 23.7g/L | 0g/L |
| Threonine | 0g/L | 4.7g/L |

As described above, when the β-fructofuranosidase gene of the present invention was introduced into a microorganism incapable of using sucrose as a carbon source, the microorganism had have the ability to metabolize sucrose and, in addition, could produce various metabolites, including acetic acid, formic acid, lactic acid, succinic acid and ethanol, using sucrose as a carbon source. Moreover, when the gene of the present invention was introduced into the metabolically modified strain in the same manner, a desired metabolite (threonine in this Example) could be successfully produced. In addition, any person skilled in the art can also easily apply the present invention to produce biodegradable polymers, recombinant proteins and the like.

### INDUSTRIAL APPLICABILITY

As described above in detail, the present invention provides a recombinant microorganism capable of using inexpensive sucrose as a carbon source instead of expensive glucose. Also, in a process of culturing microorganisms which have been incapable of using sucrose as a carbon source, sucrose can substitute for other carbon sources including glucose. In the present invention, a group of novel enzymes enabling the efficient use of sucrose which is inexpensive and abundant in nature was identified and developed. Such enzymes will be used for the more efficient and economical production of useful chemical compounds or medical recombinant proteins through microbial fermentation using sucrose as a carbon source. Particularly, because sucrose is known to function to prevent modification of proteins in cells, stabilize proteins in cells and minimize the lysis of cells, it is highly useful in the production of high concentrations of basic chemical compounds or in high-concentration cell culture.

Although the present invention has been described in detail with reference to the specific features, it will be apparent to those skilled in the art that this description is only for a preferred embodiment and does not limit the scope of the present invention. Thus, the substantial scope of the present invention will be defined by the appended claims.

## Claims

1. A recombinant vector containing a gene (*ptsG*) encoding a sucrose phosphotransferase and a *sacC* gene encoding sucrose-6-phosphate hydrolase, wherein the *ptsG* gene has SEQ ID NO: 2.

2. The recombinant vector according to claim 1, wherein the *sacC* gene has SEQ ID NO: 4.

3. A recombinant microorganism capable of metabolizing sucrose in which the recombinant vector of claim 1 is introduced into a host cell selected from the group consisting of bacteria, yeast and fungi.

4. A recombinant microorganism capable of metabolizing sucrose in which a gene (*ptsG*) encoding a sucrose phosphotransferase and a gene (*sacC*) encoding sucrose-6-phosphate hydrolase is introduced into a chromosomal DNA of a host cell selected from the group consisting of bacteria, yeast and fungi, wherein the *ptsG* gene has SEQ ID NO: 2.

5. The recombinant microorganism capable of metabolizing sucrose according to claim 4, wherein the *sacC* gene has SEQ ID NO: 4.

6. A method for producing metabolites, biodegradable polymers or recombinant proteins, the method comprises culturing the recombinant microorganism of claim 3 in a medium containing sucrose as a carbon source.

7. A method for producing metabolites, biodegradable polymers or recombinant proteins, the method comprises culturing the recombinant microorganism of claim 4 in a medium containing sucrose as a carbon source.

## Patentansprüche

1. Rekombinanter Vektor, der ein Gen (*ptsG*), das eine Saccharose-Phosphotransferase codiert, und ein *sacC*-Gen, das Saccharose-6-Phosphat-Hydrolase codiert, enthält, wobei das *ptsG*-Gen SEQ ID NO: 2 hat.

2. Rekombinanter Vektor gemäß Anspruch 1, wobei das *sacC*-Gen SEQ ID NO: 4 hat.

3. Rekombinanter Mikroorganismus, der dazu in der Lage ist, Saccharose zu metabolisieren, wobei der rekombinante Vektor gemäß Anspruch 1 in eine Wirtszelle, ausgewählt aus der Gruppe, bestehend aus Bakterien, Hefe und Pilzen, eingeführt ist.

4. Rekombinanter Mikroorganismus, der dazu in der Lage ist, Saccharose zu metabolisieren, wobei ein Gen *(ptsG),* das eine Saccharose-Phosphotransferase codiert und ein Gen (*sacC*), das Saccharose-6-Phosphat-Hydrolase codiert, in eine chromosomale DNA einer Wirtszelle, ausgewählt aus der Gruppe, bestehend aus Bakterien, Hefe und Pilzen, eingeführt ist, wobei das *ptsG*-Gen SEQ ID NO: 2 hat.

5. Rekombinanter Mikroorganismus, der dazu in der Lage ist, Saccharose zu metabolisieren, gemäß Anspruch 4, wobei das sacC-Gen SEQ ID NO: 4 hat.

6. Verfahren zur Herstellung von Metaboliten, bioabbaubaren Polymeren oder rekombinanten Proteinen, wobei das Verfahren das Kultivieren des rekombinanten Mikroorganismus gemäß Anspruch 3 in einem Medium, das Saccharose als Kohlenstoffquelle enthält, umfasst.

7. Verfahren zur Herstellung von Metaboliten, bioabbaubaren Polymeren oder rekombinanten Proteinen, wobei das Verfahren das Kultivieren des rekombinanten Mikroorganismus gemäß Anspruch 4 in einem Medium, das Saccharose als Kohlenstoffquelle enthält, umfasst.

## Revendications

1. Vecteur recombinant contenant un gène *(ptsG)* codant pour une saccharose phosphotransférase et un gène *sacC* codant pour une saccharose-6-phosphate hydrolase, où le gène *ptsG* a la SEQ ID NO : 2.

2. Vecteur recombinant selon la revendication 1, où le gène *sacC* a la SEQ ID NO : 4.

3. Micro-organisme recombinant capable de métaboliser le saccharose dans lequel le vecteur recombinant selon la revendication 1 est introduit dans une cellule hôte choisie dans le groupe constitué par des bactéries, des levures et des champignons.

4. Micro-organisme recombinant capable de métaboliser le saccharose dans lequel un gène *(ptsG)* codant pour une saccharose phosphotransférase et un gène *sacC* codant pour une saccharose-6-phosphate hydrolase sont introduits dans un ADN chromosomique d'une cellule hôte choisie dans le groupe constitué par des bactéries, des levures et des champignons, où le gène *ptsG* a la SEQ ID NO : 2.

5. Micro-organisme recombinant capable de métaboliser le saccharose selon la revendication 4, où le gène *sacC* a la SEQ ID NO : 4.

6. Procédé de production de métabolites, de polymères biodégradables ou de protéines recombinantes, le procédé comprenant la culture du micro-organisme recombinant selon la revendication 3 dans un milieu contenant du saccharose en tant que source de carbone.

7. Procédé de production de métabolites, de polymères biodégradables ou de protéines recombinantes, le procédé comprenant la culture du micro-organisme selon la revendication 4 dans un milieu contenant du saccharose en tant que source de carbone.
